# EUROPEAN PATENT SPECIFICATION

(11) **EP 1 328 643 B1**
(45) Date of publication and mention of the grant of the patent: **06.09.2006**
(21) Application number: 01982940.7
(22) Date of filing: 22.10.2001
(51) Int. Cl.: C12N 15/53, C12N 15/54, C12N 9/02, C12N 9/04, C12N 9/10, C12P 13/04, C12P 41/00, C12N 1/21, C12N 15/63

(54) **FERMENTATIVE PRODUCTION OF D-p-HYDROXYPHENYLGLYCINE AND D-PHENYLGLYCINE**
FERMENTATIVE HERSTELLUNG VON D-P-HYDROXYPHENYLGLYCIN UND D-PHENYLGLYCIN
PRODUCTION PAR FERMENTATION DE D-P-HYDROXYPHENYLGLYCINE ET DE D-PHENYLGLYCINE

(30) Priority: 27.10.2000 US 697769
(43) Date of publication of application: 23.07.2003
(73) Proprietor: DSM IP Assets B.V., 6411 TE Heerlen (NL); DSM Biotech GmbH, 52428 Jülich (DE); JOHNS HOPKINS UNIVERSITY, Baltimore, MD 21205 (US)
(72) Inventor: TOWNSEND, Craig, A., Baltimore, MD 21212 (US); GUNSIOR, Michele, Wyoming, PA 18644 (US); MÜLLER, Ulrike, 52441 Linnich-Rurdorf (DE); ASSEMA VAN, Friso, Bernard, Jan, NL-6166 XK Geleen (NL); SONKE, Theodorus, NL-6143 BK Sittard (NL)
(74) Representative: Scheltus, Irma
(86) International application number: PCT/NL2001/000772
(87) International publication number: WO 2002/034921

(56) References cited:
- SRIUBOLMAS N ET AL.: "Microbial sources of bioactive compounds and enzymes useful for antibiotic industry" BIOTECHNOLOGY FOR SUSTAINABLE UTILIZATION OF BIOLOGICAL RESOURCES IN THE TROPICS, vol. 14, 2000, pages 165-182, XP001100043
- WIYAKRUTTA S ET AL.: "A stereo-inverting D-phenylglycine aminotransferase from Pseudomonas stutzeri ST-201: purification, characterization and application for D-phenylglycine synthesis" JOURNAL OF BIOTECHNOLOGY, vol. 55, no. 3, 4 July 1997 (1997-07-04), pages 193-203, XP004126072 ISSN: 0168-1656 cited in the application
- HERMES H F M ET AL: "Metabolism of amino acid amides in Pseudomonas putida ATCC 12633." APPLIED MICROBIOLOGY AND BIOTECHNOLOGY, vol. 40, no. 4, 1993, pages 519-525, XP008007321 ISSN: 0175-7598
- TOWNSEND C A ET AL.: "Biosynthetic studies of nocardicin A" JOURNAL OF THE AMERICAN CHEMICAL SOCIETY, vol. 103, no. 10, 1981, pages 2873-2874, XP002211179
- CHOROBA O W ET AL.: "Biosynthesis of the vancomycin group of antibiotics: Involvement of an unusual dioxygenase in the pathway to (S)-4-hydroxyphenylglycine" JOURNAL OF THE AMERICAN CHEMICAL SOCIETY, vol. 122, no. 22, 7 June 2000 (2000-06-07), pages 5389-5390, XP002211180 cited in the application
- DATABASE EMBL [Online] EMBL; 13 February 1998 (1998-02-13) KERSHAW J: "Amycolatopsis orientalis cosmid PCZA361" Database accession no. AJ223998 XP002211182 -& VAN WAGENINGEN A M A ET AL.: "Sequencing and analysis of genes involved in the biosynthesis of a vancomycin group antibiotic" CHEMISTRY AND BIOLOGY, vol. 5, no. 3, March 1998 (1998-03), pages 155-162, XP000915583
- KRINGS U ET AL.: "Degradation of [2H]phenylalanine by the basidiomycete Ischnoderma benzoinum" JOURNAL OF BIOTECHNOLOGY, vol. 51, no. 2, 1 November 1996 (1996-11-01), pages 123-129, XP004037116 ISSN: 0168-1656 cited in the application
- TAYLOR P P ET AL.: "Novel biosynthetic approaches to the production of unnatural amino acids using transaminases" TRENDS IN BIOTECHNOLOGY, vol. 16, no. 10, 1 October 1998 (1998-10-01), pages 412-418, XP004145648 ISSN: 0167-7799 cited in the application
- BERRY A: "Improving production of aromatic compounds in Escherichia coli by metabolic engineering" TRENDS IN BIOTECHNOLOGY, vol. 14, no. 7, 1 July 1996 (1996-07-01), pages 250-256, XP004035764 ISSN: 0167-7799 cited in the application
- HUBBARD B K ET AL.: "Biosynthesis of L-p-hydroxyphenylglycine, a non-proteinogenic amino acid constituent of peptide antibiotics." CHEMISTRY & BIOLOGY, vol. 7, no. 12, December 2000 (2000-12), pages 931-942, XP002211181 ISSN: 1074-5521
- J CHEM SOC, CHEM COMMUN, 1982, pages 344-346,

## Description

### Field of the invention

The invention relates to a biochemical process for the preparation of D-*p*-hydroxyphenylglycine (D-HPG) and D-phenylglycine (D-PG) in enantiomerically pure form. The invention also relates to recombinant microorganisms for the production of D-HPG and D-PG. Hereinafter the abbreviation (H)PG refers to HPG and/or PG; where required the specific enantiomer of (H)PG is mentioned. This invention was made with German Government support under Grant No.0311644 awarded by the BioRegio program of the Bundesministerium für Bildung und Forschung (BMBF), and with U.S. Government support under Grant No. R01 AI14937 awarded by the National Institutes of Health. The U.S. Government has certain rights in this invention.

### Background of the invention

Except for glycine, each of the common, naturally occurring amino acids can exist as one of two possible enantiomers. The two enantiomeric forms of an amino acid can be referred to as the D- and the L-configured enantiomer. The enantiomerical purity of compounds which may exist in two enantiomeric forms is generally expressed in terms of its enantiomeric excess (often abbreviated as "e.e."); e.e. can be defined as the difference in the amounts of the two enantiomers divided by the sum of those amounts, the quotient being multiplied by 100 to get a percentage value. In the context of this application enantiomerically pure will mean an e.e. of at least 90%, preferably more than 95%, or even more than 98%.

The distinction between the D- and the L-configured enantiomer is made upon whether the conformation of the α-carbon of the amino acid corresponds to the L- or D-form of glyceraldehyde, an arbitrary standard. Most enzymes which act upon amino acids have L-specific binding domains, thus, most naturally occurring proteins comprise only L-amino acids.

There are, however, a few exceptions in which D-amino acids are used by and built into microbial cells. Bacterial cells, for example, produce D-glutamate and D-alanine as a precursor material for murein, a typical cell wall component. D-amino acids are typically not produced directly, but rather by conversion of the L-amino acid to the corresponding D-amino acid by the use of an amino acid specific racemase. Racemases catalyze the conversion of L-amino acids to D-amino acids and vice versa. In equilibrium, one finds a racemic (50 %/50 %) mixture of L- and D-enantiomers, hence, racemases cannot be used as a final reaction step for the production of D-amino acids in enantiomerically pure form.

An increasing demand for antibacterial drugs has led to a mass production of semisynthetic antibiotics, many of which have incorporated optically active D-amino acids as building blocks. D-HPG and D-PG, e.g., are incorporated in some β-lactam antibiotics, such as amoxicillin and cephalexin.

For cost effective production of these antibiotics, it is required to have available a commercially attractive process for the preparation of D-(H)PG in enantiomerically pure form. However, due to their complex structure and due to the occurrence of a chiral center within the molecules, the production of D-(H)PG with conventional chemical methods generally involves multiple steps for the production of a racemic mixture of D- and L-configured (H)PG and then requires additional optical resolution steps to obtain the products in enantiomerically pure form. The additional process steps add to the process costs and render the overall process commercially less attractive.

Fermentative processes for the production of fine chemicals are generally known as being commercially attractive for their ability to convert relatively cheap starting material to the product. The reactions involved in fermentative processes are usually highly regio- and stereoselective. They therefore allow the production of complex and enantiomerically pure products in relatively simple processes. For this reason, numerous attempts have been made in the prior art to produce various fine chemicals by fermentation.

Methods for the production of D-phenylalanine in a fermentative process using an *E. coli* strain containing a *Bacillus sphaericus* stereo-conserving D-aminotransferase, have been described (EP 0736604A2). "Stereo-conserving D-aminotransferases" shall be understood as D-aminotransferases that exclusively produce D-configured amino acids from D-configured amino donors. "Stereo-inverting D-aminotransferases", on the other hand, shall be defined as aminotransferases which use L-configured amino donors and exclusively produce D-configured amino acids. For instance, L-Glu or L-Asp can be used as amino donor in the production of D-PG or D-HPG.

In order to provide the D-configured amino donor for the stereo-conserving D-specific aminotransferase reaction *in vivo,* genes coding for racemases which catalyze the conversion of L-amino acid to D-amino acid and vice versa, must be present in the cell. Hence, the D-configured amino donor is produced intracellularly from the L-configured enantiomer. The intracellular concentration of the D-configured amino donor will be half or less than half of the total intracellular concentration of said molecule (D- plus L-form). Because decreasing concentrations of the D-configured amino donor (an educt) lead to a shift of the equilibrium of the transamination reaction towards the educt side, a racemic mixture of the amino donor (i.e. the use of racemases in combination with stereo-conserving aminotransferases) is unfavorable for the efficient production of the desired D-amino acid.

Additionally, cloning a racemase into the cell is costly in labor and it increases the "metabolic burden" of the cell in that the cell has to synthesize an additional enzyme. The precursor metabolites and the energy consumed for the biosynthesis of the racemase are no longer available for cell growth, product formation, etc.

The aim of the current invention is to make available an improved fermentative process for the production of a D-phenylglycine product, in particular D-phenylglycine (D-PG) or D-*p*-hydroxyphenylglycine, which overcomes the above mentioned shortcomings.

This aim, surprisingly, is achieved in that in the fermentative process
a) for the production of D-PG, respectively for the production of D-HPG, phenylpyruvate (PP), respectively *p*-hydroxyphenylpyruvate (HPP) is withdrawn from the aromatic amino acid pathway
b) and is converted to mandelic acid (MA) or *p*-hydroxymandelic acid (HMA), respectively,
c) thereafter being converted into phenylglyoxylate or *p*-hydroxyphenylglyoxylate, respectively,
d) and the phenylglyoxylate or *p*-hydroxyphenylglyoxylate thereafter being converted into D-phenylglycine (D-PG) or D-*p*-hydroxyphenylglycine (D-HPG) by the action of a stereo-inverting D-aminotransferase, respectively,

The application of a stereo-inverting aminotransferase in a fermentative process provides the clear advantages that no racemase activity is required and that the stereo-inverting aminotransferase uses naturally occurring L-configured amino donors (which are present intracellularly in at least twice the concentration of the respective D-enantiomer, when said D-enantiomer was produced from the L-enantiomer by a racemase). The application of a fermentative process allows the utilization of cheap starting materials such as glucose and other sugars for the production of D-(H)PG.

Fermentation (and a fermentative process), within the meaning of the invention, shall be understood as the process of cultivating a microorganism in a suitable medium, converting components of a suitable medium to the product by the catalytic activity of cellular components or by spontaneous chemical reactions, and obtaining the product from the cultivation broth and/or from the biomass itself.

A suitable medium, within the meaning of the invention, shall be understood as a mixture of substances on which the microorganism can grow, proliferate, and/or which can be converted to the desired product.

In a preferred embodiment of the invention, the microorganism applied in the fermentation is selected from a group of microorganisms which replenish HPP and/or PP (in cases where HPP and/or PP are withdrawn from the respective metabolite pool) more efficiently than typically observed in wild type strains. A microorganism selected from said group of microorganisms shall be referred to as having "improved production capabilities" for HPP and/or PP, and to "provide HPP and/or PP at increased availability".

The application of a microorganism that provides HPP and/or PP at increased availability is important for the efficient production of D-HPG or D-PG because in a sequence of reactions not only the slowest reaction of the sequence but every enzymatic reaction controls the production rate to a certain extent. Hence, also the conversion from glucose to HPP and/or PP has an influence on the total production of the final product D-HPG or D-PG.

Suitable measures to increase the availability of HPP and PP in said microorganisms comprise the selection of microorganisms showing a beneficial spontaneous mutation, the selection of microorganisms that evolve from classical strain improvement programs involving random mutagenesis, and the selection of microorganisms with improved production capabilities that evolved from application of recombinant DNA technology. Suitable measures to increase the availability of HPP and PP, e.g., can be the introduction of feedback resistant mutants of key enzymes or the overexpression of one or several pathway enzymes as reviewed by Berry (1996, TIBTECH, 14: 250-256). Application of recombinant DNA technology can also be directed towards the introduction of phosphoenolpyruvate-independent sugar uptake systems (WO 98/18936), towards the deletion of the phosphotransferase system (PTS) (Berry, 1996, TIBTECH, 14: 250-256) and/or towards an increased availability of phosphoenolpyruvate by modifications of reactions acting on the intracellular phosphoenolpyruvate concentration (Berry, 1996, TIBTECH, 14: 250-256). Application of recombinant DNA technology can also be directed towards introducing increased intracellular transketolase and/or transaldolase activity (WO 98/18936).

Another suitable measure to provide HPP and PP at increased availability is to supply L-tyrosine, respectively L-phenylalanine, in the fermentation medium. L-Tyrosine is readily converted to HPP, whereas L-phenylalanine is readily converted to PP, by the microorganism in a single aminotransferase reaction step.

In a preferred embodiment, microorganisms according to the invention, convert PP to MA and/or convert HPP to HMA in a single-step enzymatic conversion catalyzed by *p*-hydroxymandelate synthase (*p*-HmaS). The conversion of HPP to HMA by an enzyme named *p*-HmaS was described by Choroba et al. (2000, J. Am. Chem. Soc., 122 (22): 5389-90).

The advantage of using a single enzyme for the conversion of HPP or PP to HMA or MA is that it is usually easier to clone and establish the *in vivo* activity of a single enzyme as compared to cloning and establishing the activity of multiple enzymes performing the same overall conversion.

Alternatively, a microorganism according to the invention converts PP to MA and/or HPP to HMA in a sequence of reactions comprising the reactions from PP or HPP for instance to phenylacetaldehyde or *p*-hydroxyphenylacetaldehyde to phenylacetate or *p*-hydroxyphenylacetate to MA or HMA, respectively. This sequence of reactions is deemed a possible metabolic route as it was observed in the degradation of phenylalanine in a basidiomycete by Krings et al. (1996, Journal of Biotechnology, 51: 123-129)).

Microorganisms according to the invention, irrespective of the way in which they produce HMA or MA, convert MA to PGL and/or HMA to HPGL by the enzymatic activity of a mandelate dehydrogenase and/or a *p*-hydroxymandelate dehydrogenase, respectively. Alternatively, said microorganisms convert MA to PGL and/or HMA to HPGL by the enzymatic activity of an oxygen dependent mandelate oxidase and/or an oxygen dependent *p*-hydroxymandelate oxidase, respectively. Microorganisms according to the invention are furthermore capable of converting HPGL, respectively PGL, to D-HPG, respectively to D-PG, by the action of a stereo-inverting aminotransferase.

The present invention also relates to the production of D-(H)PG by culturing said recombinant cells in a suitable medium and obtaining the product from the culture broth or from the microorganism itself.

The present invention also relates to a recombinant microorganism (i.e. to a recombinant cell) which is capable of secreting detectible amounts of D-HPG and/or D-PG and contains genes, coding for enzymes which catalyze in a single step, and/or by combined and subsequent action, the conversion of HPP to HMA and/or of PP to MA, the conversion of HMA to HPGL and/or of MA to PGL, and the conversion of HPGL to D-HPG and/or of PGL to D-PG, the final step being catalyzed by the action of a stereo-inverting D-aminotransferase.

It is to be noticed that the production of D-PG and D-HPG using a stereo-inverting aminotransferase is described by Wiyakrutta and Meevootisom (1997, Journal of Biotechnology, 55: 193-203). However, a whole cell fermentative approach is not considered. Instead, the authors mention reactions with purified enzyme. Furthermore, Taylor et al. (1998, TIBTECH, 16: 412-418) mention that the educts, PGL and HPGL, are very expensive, as compared to the value of the final products, D-PG and D-HPG. Thus, these references do not suggest any feasible application of a stereo-inverting aminotransferase for the production of D-HPG or D-PG.

Moreover, combination of a fermentative pathway to HPGL or PGL with the enzymatic activity of a stereo-inverting D-aminotransferase *in vivo* would have been expected to have limited chances of success because of incompatible pH optima of the respective enzymes. The intracellular pH of *E. coli* cells is reported to be in the range of pH 7.4 to pH 7.8 (Neidhardt et al., 1996, *Escherichia coli* and Salmonella *typhimurium*. Cellular and molecular biology., volume 1, chapter 96, page 1539, ASM Press, Washington, D.C.). The stereo-inverting aminotransferases, however, as reported by Wiyakrutta and Meevootisom (see above) have pH optima at pH 9 to pH 10 and very little activity at neutral pH. Thus, one would expect that a stereo-inverting aminotransferase does not have sufficient *in vivo* activity in *E. coli* cells to produce D-HPG or D-PG effectively.

Apart from an incompatibility of pH optima, several other problems can be expected, when enzymes from different organisms are combined to a new metabolic pathway in a host cell. The affinity of newly introduced enzymes might be very different from the intracellular metabolite concentrations. Furthermore, the stability of the heterologous enzyme might be low due to a high susceptibility of heterologous proteins towards intracellular proteases. The intermediates of the newly constructed pathway themselves might be unstable or metabolized by native enzymes of the host cell. Problems in the functional expression of the heterologous enzymes can also arise from inappropriate folding of correctly translated amino acid chains or from a difference in codon usage which might hinder the effective functional expression of the heterologous enzyme.

Despite the numerous problems that are expected in the construction of the new metabolic pathway, surprisingly, we found that D-HPG and D-PG were produced by recombinant microorganisms in a process according to the invention.

Other features of the invention will become apparent in the course of the following descriptions of the examples.

### General procedures

Standard molecular cloning techniques such as plasmid DNA isolation, gel electrophoresis, enzymatic restriction modification of nucleic acids, *E. coli* transformation etc. were performed as described by Sambrook et al., 1989, "Molecular Cloning: a laboratory manual", Cold spring Harbor Laboratories, Cold Spring Harbor, NY. Synthetic oligodeoxynucleotides were obtained from MWG-Biotech AG (Ebersberg, Germany), Sigma-Genosys (The Woodlands, TX, USA), and LifeTechnologies (Paisley, Scotland, UK). DNA sequence analyses were performed by BaseClear (Leiden, The Netherlands), GATC Biotech AG (Konstanz, Germany), and Johns Hopkins University School of Medicine Biosynthesis and Sequencing Facility (Baltimore, MD, USA) using the chain termination method with dye-labeled dideoxy-terminators. Protein concentrations in crude extracts were determined with Bradford reagent at 595 nm (Roth, Karlsruhe, Germany) according to the instructions of the supplier.

### Experimental Part I (Examples 1-19): Cloning and verification of encoded activity of relevant single genes

### Example 1: Construction of plasmids pBAD-Ao-HmaS, pBAD-Ao-HmaO, pBAD-Sc-HmaS, pBAD-Sc-HmaO

*Amycolatopsis orientalis* NRRL 18098 (US patent 5,843,437) was obtained from the ARS (Agricultural Research Service) Patent Culture Collection, Peoria, Illinois, USA.

*A. orientalis* was cultivated in 1 % glucose, 0.5 % yeast extract (Difco, Detroit, Michigan, USA), 2 % starch, 0.1 % casamino acids (Difco), pH 7.5 with NaOH at 28°C. *Streptomyces* coelicolorA3(2), kindly obtained from Professor M.J. Bibb of John Innes Institute, Norwich (UK), was cultivated in YE-ME medium containing 3 g/l yeast extract (Difco), 5 g/l peptone (Difco), 3 g/l malt extract (Oxoid, Basingstoke, UK), 10 g/l glucose, 340 g/l sucrose at 28°C. 10 g/l glycine and 5 mM MgCl₂ were added after sterilisation.

The genomic DNA from *A. orientalis* and *S. coelicolor* was isolated by a salting out procedure (Pospiech and Neumann, 1995. Trends Genet. 11: 217-218).

The HmaS and HmaO genes of *S. coelicolor* and *A. orientalis* were cloned in expression vector pBAD/*Myc*-HisC (Invitrogen, Groningen, The Netherlands). Genes were cloned via a translation-start (ATG) fusion and with their original stop codons.

### 1.1 Construction of plasmid pBAD-Ao-HmaS

A 1131 bp fragment comprising the open reading frame (ORF) for *p*-hydroxymandelate synthase was amplified by PCR from the chromosomal DNA from *Amycolatopsis orientalis* NRRL18098 (nucleotides 14957-16030 of accession number AJ223998; amplified region nucleotides 14957-16060) using the following primers:
5' - GTCCACGGTCTCCCATGCAGAATTTCGAGAT - 3' [SEQ ID: No.1]
   (with *Bsa* I recognition and cleavage site underlined), and
5' - ACATCCCAAGCTTCACGTTCGAGGTC - 3' [SEQ ID: No.2]
   (with *Hind* III cleavage site underlined).

A list of all oligonucleotide sequences used in the context of the present application is presented in Annexe 4 hereto.

Correct size of the amplified fragment was confirmed by agarose gel electrophoresis and the fragment was purified from the gel. The fragment was digested with the enzymes *Bsa* I and *Hin*d III to generate sticky ends. The plasmid pBAD/*Myc*-HisC was digested with *Nco* I and *Hin*d III. The two fragments were subsequently ligated and used for the transformation of chemically competent cells of *E. coli* Top10 (Invitrogen, Groningen, The Netherlands). The transformants were selected on LB agar plates containing 100 mg/l ampicillin. A plasmid showing the correct insert sequence (as confirmed by sequencing) was given the name pBAD-Ao-HmaS, which has been deposited under the Budapest Treaty at the Deutsche Sammlung für Mikroorganismen und Zellkulturen, Braunschweig, Germany (DSMZ), on October 23^{rd}, 2000, under deposit number DSM 13786, and was used for further investigations.

### 1.2 Construction of plasmid pBAD-Ao-HmaO

A 1096 bp fragment comprising the ORF for the *p*-hydroxymandelate oxidase was amplified by PCR from *Amycolatopsis orientalis* NRRL18098 chromosomal DNA (nucleotides 16027-17100 of accession number AJ223998; amplified region nucleotides 16027-17101) using the following primers:
5' - CGCTCGGTCATGACGTACGTTTCCCTG - 3' [SEQ ID: No.3]
   (with BspH I cleavage site underlined), and
5' - ACGAAGAAGCTTATCAAACAACCCCCAG - 3' [SEQ ID: No.4]
   (with *Hind* III cleavage site underlined).

Correct size of the amplified fragment was confirmed by agarose gel electrophoresis and the fragment was purified from the gel.

The fragment was digested with the enzymes *BspH* I and *Hind* III and the plasmid pBAD/*Myc*-HisC was digested with *Nco* I and *Hin*d III. The two fragments were ligated and introduced into *E. coli* Top10 cells. The transformants were selected on LB agar plates containing 100 mg/l ampicillin. A plasmid showing the correct insert sequence (as confirmed by sequencing) was called pBAD-Ao-HmaO which has been deposited under the Budapest Treaty at the DSMZ on October 23^{rd}, 2000, under deposit number DSM 13791, and was used for further investigations.

### 1.3 Construction of plasmid pBAD-Sc-HmaS

A 1091 bp fragment comprising the ORF for the *p*-hydroxymandelate synthase was amplified by PCR from *Streptomyces coelicolor* A3(2) strain M145 chromosomal DNA (encoded by nucleotides 1418-2533 of accession AL035640; amplified region nucleotides 1415-2494) using the following primers:
5' - ATGCCGCCCAGTGACATCGCGTACGC - 3' [SEQ ID: No.5]
   and
5' - CCCTCGGTACCAGGTCATCGGCCGGCCACTTCC - 3' [SEQ ID: No.6]
   (with *Kpn* I restriction site underlined).

Correct size of the amplified fragment was confirmed by agarose gel electrophoresis and the fragment was purified from the gel. The amplified fragment was cloned into the *Nco* I/*Kpn* I site of the vector pBAD/*Myc*-HisC as described by Dietmaier et al. (1993, Nucleic Acids Res. 21, 3603-3604).

The resulting plasmid was introduced into *E. coli* Top10 cells by electroporation. The transformants were selected on LB agar plates containing 100 mg/l carbenicillin.

All colonies resulting from the transformation were collected and the resulting cell suspension was used for plasmid DNA isolation. The total plasmid DNA was digested with *Hind* III and separated on an agarose gel. A 5 kb DNA fragment being the target plasmid in linear form was isolated from the gel, re-ligated and introduced into chemically competent *E. coli* Top10 cells by electroporation.

The transformants were selected on LB agar plates containing 100 mg/l carbenicillin.

A plasmid showing the correct insert sequence (as confirmed by sequencing) was given the name pBAD-Sc-HmaS which has been deposited under the Budapest Treaty at the DSMZ on October 23^{rd}, 2000, under deposit number DSM 13790, and was used for further investigations.

### 1.4 Construction of plasmid pBAD-Sc-HmaO

A 1149 bp fragment comprising the ORF for the *p*-hydroxymandelate oxidase was amplified by PCR from the *Streptomyces coelicolor* A3(2) strain M145 chromosomal DNA (nucleotides 135-1268 of accession AL035640; amplified region nucleotides 132-1267) using the following primers:
5' - ATGCGGGAGCCGCTCACGCTCGAC - 3' [SEQ ID: No.7]
   and
5' - CCAACTGGTACCTGGTCATCCGTGGCTCCTGTCTCG - 3' [SEQ ID: No.8]
   (with *Kpn* I restriction site underlined).

Correct size of the amplified fragment was confirmed by agarose gel electrophoresis and the fragment was purified from the gel. The amplified fragment was cloned into the *Nco* I/*Kpn* I site of vector pBAD/*Myc*-HisC as described by Dietmaier et al. (1993, Nucleic Acids Res. 21, 3603-3604) and the resulting plasmid was subsequently introduced into *E. coli* Top10 cells. Transformants were selected on LB agar plates containing 100 mg/l carbenicillin.

Plasmid DNA was isolated from all transformants, digested with *Hind* III and separated on an agarose gel. A 5.2 kb DNA fragment being the target plasmid in linear form was isolated from the gel, re-ligated and introduced into chemically competent *E. coli* Top10 cells. The transformants were selected on LB agar plates containing 100 mg/l carbenicillin.

A plasmid showing the correct insert sequence (as confirmed by sequencing) was given the name pBAD-Sc-HmaO, which has been deposited under the Budapest Treaty at the DSMZ on October 23^{rd}, 2000, under deposit number DSM 13789, and was used for further investigations.

### Example 2: Expression of p-hydroxymandelate synthase and p-hydroxymandelate oxidase from A. orientalis and S. coelicolor

Single colonies of the *E. coli* Top10 strains harboring the plasmids pBAD-Ao-HmaS or pBAD-Sc-HmaS (for *p*-hydroxymandelate synthase) and the plasmids pBAD-Ao-HmaO or pBAD-Sc-HmaO (for *p*-hydroxymandelate oxidase) were cultivated in 50 ml LB medium containing 100 mg/l carbenicillin at 30°C. At OD₆₂₀ₙₘ 1.2, the cells were induced by the addition of 0.002 % (final concentration) arabinose. After 3.5 hours the cells were harvested and washed with 1 mM MgSO₄ at pH 7.4. Aliquots of washed cells were frozen at -20°C for later use. As a control, *E. coli* Top10 harboring plasmid pBAD/*Myc*-HisC was treated accordingly.

Crude extracts were prepared by sonification in 200 mM potassium phosphate buffer pH 7.5 immediately before use.

### Example 3: Analysis of p-hydroxymandelate synthase from A. orientalis and S. coelicolor

### 3.1 Activity towards p-hydroxyphenylpyruvate

The assay mixture of 3 ml contained 200 mM potassium phosphate buffer pH 7.5, 5 mM *p*-hydroxyphenylpyruvate, 10 % ethanol (50 mM *p*-hydroxyphenylpyruvate stock solution in 96 % ethanol was used), 44 mM ascorbate, 0.3 mM FeSO₄, and cell free extract leading to a final concentration of 0.6 mg/ml of soluble protein. Boiled extracts were used in control experiments.

The assay was started by the addition of HPP and stopped after 1 hour at 28°C by the addition of 0.1 ml 1 N HCl to an aliquot of 0.5 ml of the reaction system. The samples were analyzed by HPLC and detected at 215 nm. A Nucleosil-120-5-C18 column (250x4 mm, Macherey-Nagel, Düren, Germany) was used. The column was eluted with eluent A (50 mM H₃PO₄) and eluent B (100 % methanol). Gradient: 0-5 min, 0 % B; 5-37 min, 0 % to 90 % B; 37-42 min, 90 % B; 42-50 min, 90 % to 0 % B; 50-55 min, 0 % B. The flow was 1.0 ml/min, the column temperature was set at 30°C.

65 mg/l *p*-hydroxymandelate was produced within 1 hour with cell free extracts derived from *E. coli*/pBAD-Ao-HmaS, while 35.6 mg/l *p*-hydroxymandelate was produced with extracts derived from *E. coli*/pBAD-Sc-HmaS. No *p*-hydroxymandelate was produced in the control experiments (crude extracts of *E. coli*/pBAD/*Myc*-HisC and boiled extract).

### 3.2 Activity towards phenylpyruvate

The assay mixture of 3 ml contained 200 mM potassium phosphate buffer pH 7.5, 5 mM phenylpyruvate, 44 mM ascorbate and 0.3 mM FeSO₄ and cell free extract at a final concentration of 0.6 mg/ml of soluble protein.

The assay was started at 28°C by the addition of the cell free extract and stopped by the addition of 0.1 ml 1 N HCl to an aliquot of 0.5 ml of the reaction system. Samples were analyzed by HPLC as described above. Both boiled extracts and the crude extract of *E. coli*/pBAD/*Myc*-HisC were used in control experiments.

63 mg/l mandelate was produced within 1 hour using cell free extract derived from *E. coli*/pBAD-Ao-HmaS. Cell free extract derived from *E. coli*/pBAD-Sc-HmaS produced 25 mg/l mandelate within 6 hours. No mandelate was detected in the control experiments.

### Example 4: Analysis of p-hydroxymandelate oxidase from A. orientalis and S. coelicolor

### 4.1 Activity towards D,L-p-hydroxymandelate

100 µl of the crude extract containing 0.5 - 0.8 mg protein was incubated with 100 mM potassium phosphate buffer pH 7.5, 2 mM D,L-*p*-hydroxymandelate, 20 mg/l catalase in a total volume of 1 ml. The oxidation of *p*-hydroxymandelate was monitored spectrophotometrically at 340 nm. To correct for non-specific oxidation of *p*-hydroxymandelate, control assays were run using an assay mixture without cell free extract.

The specific activity was 30 nmol*min⁻¹*mg⁻¹ total protein for the extracts of *E. coli* Top10/pBAD-Sc-HmaO and 5 nmol*min⁻¹*mg⁻¹ total protein for *E. coli* Top10/pBAD-Ao-HmaO.

### 4.2 Activity towards (S)- or (R)-mandelate

The assay mixture of 3 ml contained 100 mM potassium phosphate buffer pH 7.5, 2 mM (*S*)- or (*R*)-mandelate, 20 mg/l catalase and cell free extract at a final concentration of 0.6 mg/ml of soluble protein.

The assay was started by addition of the cell free extract and stopped by the addition of 0.1 ml 1 N HCl to an aliquot of 0.5 ml of the reaction system. Samples were analyzed by HPLC by the method of Example 3. Boiled extracts were used in control experiments.

No phenylglyoxylate was produced with cell free extracts derived from *E. coli*/pBAD-Ao-HmaO or *E. coli*/pBAD-Sc-HmaO using (*R*)-mandelate as a substrate. With (*S*)-Mandelate as a substrate 9 mg/l phenylglyoxylate were produced within 1 hour using cell free extract from *E. coli*/pBAD-Ao-HmaO, and 27 mg/ml phenylglyoxylate was produced within 1 hour with cell free extract derived from *E. coli*/pBAD-Sc-HmaO.

### Example 5: Construction of plasmids pMAL-Nu-HmaS and pMAL-Nu-HmaO

*Nocardia uniformis* subsp. *tsuyamanensis* ATCC 21806 was obtained from the American Type Culture Collection (Manassas, VA, USA). *N. uniformis* was cultivated and genomic DNA was isolated as described in *J. Biol. Chem.* 1998, 273, 30695-30703. Genes were cloned into the pMAL-c2 expression vector (New England BioLabs, Beverly, MA, USA) with their original stop codons resulting in a maltose-binding protein fusion protein. HmaS was also cloned into pET-29b (Novagen, Madison, WI, USA) as a C-terminal His-6 tagged fusion.

### 5.1 Construction of plasmid pMAL-Nu-HmaS

A 1052 bp fragment comprising the open reading frame (ORF) for *p*-hydroxymandelate synthase was amplified by PCR from *Nocardia uniformis* subsp. *tsuyamanensis* chromosomal DNA (nucleotides 52-1086 of SEQ. ID: No.9, encoding the protein of SEQ.ID: No.10, given in annexe 1; amplified region nucleotides 55-1089) using the following primers:
5' - AGAATTCGCGGCACAGGCAGGCAGCG - 3' [SEQ ID: No.11]
   (with *Eco*R I cleavage site underlined) and
5' - TTATAAGCTTTCAGCGCTCGGTCCGGTGGC - 3' [SEQ ID: No.12]
   (with *Hin*d III cleavage site underlined).

Correct size of the amplified fragment was confirmed by agarose gel electrophoresis and the fragment was purified from the gel. The fragment was digested with *Eco*R I and *Hind* III. The plasmid pMAL-c2 was digested with *Eco*R I and *Hind* III and the two fragments were subsequently ligated and transformed by electroporation into *E. coli* TB1 cells (New England BioLabs, Beverly, MA, USA). The transformants were selected on LB agar plates containing 100 mg/l ampicillin. A plasmid showing the correct insert sequence (as confirmed by sequencing) was given the name pMAL-Nu-HmaS, which has been deposited under the Budapest Treaty at the ATCC on October 27^{th}, 2000, under Patent Deposit Designation PTA-2639, and used for further investigations.

### 5.2 Construction of plasmid pET-Nu-HmaS

A 1051 bp fragment comprising the open reading frame (ORF) for *p*-hydroxymandelate synthase was amplified by PCR from *Nocardia uniformis* subsp. *tsuyamanensis* chromosomal DNA (nucleotides 52-1086 of SEQ. ID: No.9, encoding the protein of SEQ.ID: No.10, given in annexe 1; amplified region nucleotides 52-1086) using the following primers:
5' - TATACCATGGCGGCACAGGCAGGC - 3' [SEQ ID: No.13]
   (with Nco I cleavage site underlined)
   and
5' - TTATAAGCTTGCGCTCGGTCCGGTGGC - 3' [SEQ ID: No.14]
   (with *Hind* III cleavage site underlined).

Correct size of the amplified fragment was confirmed by agarose gel electrophoresis and the fragment was purified from the gel. The fragment was digested with *Nco* I and *Hind* III. The plasmid pET-29b was digested with *Nco* I and *Hind* III and the two fragments were subsequently ligated and transformed by electroporation into *E. coli* BL21 (DE3) cells (Novagen, Madison, WI, USA). The transformants were selected on LB agar plates containing 50 mg/l kanamycin. A plasmid showing the correct insert sequence (as confirmed by sequencing) was given the name pET-Nu-HmaS, which has been deposited under the Budapest Treaty at the ATCC on October 27^{th}, 2000, under Patent Deposit Designation PTA-2638, and used for further investigations.

### 5.3 Construction of plasmid pMAL-Nu-HmaO

A 1144 bp fragment comprising the ORF for the *p*-hydroxymandelate oxidase was amplified by PCR from *Nocardia uniformis* chromosomal DNA (nucleotides 50-1177 of SEQ. ID: No.15, encoding the protein of SEQ.ID: No.16, given in annexe 2; amplified region nucleotides 53-1180) using the following primers:
5' - AGAATTCGGCGTCCGCAACTCCGCAG - 3' [SEQ ID: No.17]
   (with *Eco*R I cleavage site underlined) and
5' - AATAAGCTTTCAGGGCGCACCTCGCC - 3' [SEQ ID: No.18]
   (with *Hind* III cleavage site underlined).

Correct size of the amplified fragment was confirmed by agarose gel electrophoresis and the fragment purified from the gel. The fragment and plasmid pMAL-c2 were digested with *Eco*R I and *Hind* III. The two fragments were ligated and transformed into *E. coli* TB1 cells by electroporation. The transformants were selected on LB agar containing 100 mg/l ampicillin. A plasmid showing the correct insert sequence (as confirmed by sequencing) was named pMAL-Nu-HmaO, deposited under the Budapest Treaty at the ATCC on October 27^{th}, 2000, under Patent Deposit Designation PTA-2637, and used for further investigations.

### Example 6: Preparation of p-hydroxymandelate synthase and p-hydroxymandelate oxidase from N. uniformis

### 6.1 Expression of p-hydroxymandelate synthase (first method) and of p-hydroxymandelate oxidase

Single colonies of the *E. coli* TB1 strains harboring the plasmids pMAL-Nu-HmaS or pMAL-Nu-HmaO were cultivated in 100 ml LB medium (+ 0.02% glucose) containing 100 mg/l ampicillin at 37°C. At OD₆₀₀ₙₘ 0.6, the cells were induced by the addition of 0.3 mM (final concentration) isopropylthiogalactoside (IPTG). After 3 hours, the cells were harvested, resuspended in buffer (20 mM Tris-HCl pH 7.4, 200 mM NaCl, 1 mM EDTA for HmaO and 20 mM Tris-HCl pH 7.5, 10 mM EDTA, 1% Triton X-100 for HmaS) and frozen at -20°C for later use. As a control *E. coli* TB1 cells containing the plasmid pMAL-c2 were treated identically.

### 6.2 Purification of p-hydroxymandelate synthase (first method) and of p-hydroxymandelate oxidase

Crude extracts of resuspended pellets were prepared by sonication immediately prior to use. Insoluble cell debris was removed from the extract by centrifugation and the resulting cell free extract applied to an amylose resin column. The desired protein was obtained in ≥ 80% purity by elution with buffer (20 mM Tris-HCl pH 7.4, 200 mM NaCl, 1 mM EDTA) containing 10 mM maltose. The partially purified extract was used in further investigations.

### 6.3 Expression of p-hydroxymandelate synthase (second method)

Single colonies of the *E. coli* BL21 (DE3) strains harboring the plasmids pET-Nu-HmaS were cultivated in 100 ml LB medium containing 50 mg/l kanamycin at 37°C. At OD₆₀₀ₙₘ 0.6, the cells were induced by the addition of 1 mM (final concentration) IPTG. After 4 hours, the cells were harvested, resuspended in buffer (50 mM sodium phosphate pH 8, 300 mM NaCl, 10 mM imidazole) and frozen at -20°C for later use.

### 6.4 Purification of p-hydroxymandelate synthase (second method)

A crude extract was prepared by sonication immediately prior to use. Insoluble cell debris was removed from the extract by centrifugation and the resulting cell free extract was batch incubated with Ni-NTA resin (Qiagen, Valencia, CA, USA) for 1 hour at 200 rpm. The CFE-resin slurry was poured into a column and the desired protein was obtained in ≥ 90% purity by elution with buffer (50 mM sodium phosphate pH 8, 300 mM NaCl) containing increasing concentrations of imidazole. The partially purified extract was used in further investigations.

### Example 7: Activity of p-hydroxymandelate synthase from N. uniformis

The assay mixture of 0.4 ml contained 50 mM Tris-HCl buffer pH 8.0, 5 mM *p*-hydroxyphenylpyruvate, 0.7% ethanol (50 mg/ml *p*-hydroxyphenylpyruvate stock solution was used), 0.5 mM dithiothreitol (DTT), 0.1 mM ascorbate, 0.025 mM FeSO₄, and partially purified HmaS. The assay was started by addition of HmaS and stopped after 1 hour at 30°C by heat inactivation at 100°C, 5 min. Denatured protein was removed by centrifugation and 0.35 ml of the reaction system used in a coupled assay with HmaO. The assay was started with addition of 0.1 mg/ml HmaO and an increase in absorbance at 332 nm was observed due to the oxidaton of *p*-hydroxymandelate to HPGL. No HPGL was produced in control experiments.

### Example 8: Activity of p-hydroxymandelate oxidase from N. uniformis

The assay mixture of 0.5 ml contained 50 mM Tris-HCl buffer pH 8 and 2 mM (*R, S*)-*p*-hydroxymandelic acid. The assay was initiated with the addition of partially purified HmaO at a final concentration of 0.1 mg/ml. Formation of *p*-hydroxyphenylglyoxylate (HPGL) was monitored spectrophotometrically at 332 nm. No formation of HPGL was observed in the absence of substrate. The specific activity for HmaO was determined to be 240 µmol*min⁻¹*mg⁻¹.

In separate experiments with wild-type enzyme purified from *N. uniformis,* the oxidase was shown to be flavin-dependent and specific for (*S*)-mandelic acid.

### Example 9: Construction of plasmids pGEM-mdIB and pGEM-Bldm

*Pseudomonas putida* ATCC 12633 was obtained from the American Type Culture Collection (Manassas, VA, USA). *P. putida* was cultivated in LB medium (10 g/l Tryptone Peptone (Difco), 5 g/l yeast extract (Difco), 5 g/l NaCl) at 28°C. The genomic DNA from this *P. putida* strain was isolated after overnight cultivation using the standard protocol described by Ausubel et al. (1990, *Current Protocols in Molecular Biology,* chapter 2.4.3, step 1 to 9, Whiley-Interscience, New York). The crude chromosomal DNA was treated with RNAse (20 mg/l), and subsequently with phenol/chloroform/isoamyl alcohol (25:24:1) to remove proteins.

A 1409 bp fragment comprising the ORF for the (S)-mandelate dehydrogenase was amplified by PCR from the chromosomal DNA from *Pseudomonas putida* ATCC 12633 (encoded by nucleotides 2251-3432 of accession J05293; amplified region nucleotides 2110-3518) using Taq DNA-polymerase and the following primers:
5' - ACTCGCCAAGGGCTATGGTGTCC - 3' [SEQ ID: No.19]
   and
5' - GCCAACAGTTCCAACAGCGGTGTG - 3'. [SEQ ID: No.20]

Correct size of the amplified fragment was confirmed by agarose gel electrophoresis.

The amplified fragment was cloned in vector pGEM-T (Promega, Madison, Wisconsin, USA) and *E. coli* XL1-Blue MRF' (Stratagene, La Jolla, CA, USA) was transformed therewith. The transformants were selected on LB agar plates containing 100 mg/l ampicillin.

Sequencing of the insert of four different clones revealed that all clones had GC instead of CG at position 2291/2292. One of the clones, without additional mutations, and named pGEM-Bldm, has been deposited under the Budapest Treaty at the DSMZ on October 23^{rd}, 2000, under deposit number DSM 13787. This original deposit, which, wrongfully, has been made under the name pGEM-mdlB but correctly should have been named pGEM-Bldm, was used in further cloning experiments. Another clone, with an additional silent mutation, named pGEM-mdIB, was used to demonstrate the desired activity. It is noted, that in said latter plasmid the orientation of the mandelate dehydrogenase gene matches the orientation of the vector-borne *lac* promotor.

### Example 10: Expression of (S)-mandelate dehydrogenase from P. putida

*E. coli* XL1-MRF'/pGEM-mdlB was cultured in LB medium containing 100 mg/l carbenicillin and 1 mM IPTG. After 16 hours of cultivation at 37°C, cells were harvested by centrifugation and re-suspended in 50 mM potassium phosphate buffer, pH 6.8. Cells were disintegrated by ultrasonic treatment and non-broken cells and cell fragments were removed by centrifugation (5.000 g, 10 min, 4°C). Finally, membranes were collected by an ultra centrifugation step (1 hour, 200.000 g, at 4°C) and then re-suspended in 4 ml of 50 mM potassium phosphate buffer, pH 6.8 and kept on ice.

The protein concentration of the membrane fraction was determined by a modified Lowry protein assay (Sandermann and Strominger, 1972, J. Biol. Chem. 247: 5123-5131), in which 1 % of SDS is added to the Lowry reagent A. The membrane fraction contained about 1 mg/ml protein.

### Example 11: Proof of activity of the (S)-mandelate dehydrogenase

The (*S*)-mandelate dehydrogenase activity was spectrophotometrically determined using 2,6-dichlorophenol-indophenol as acceptor dye by the method of Hegeman (1966, J. Bacteriol. 91: 1140-1154). Stereoselectivity of (*S*)-mandelate dehydrogenase was examined by using (*S*)- or (*R*)-mandelate as a substrate. Further it was analyzed, whether the (*S*)-mandelate dehydrogenase can also take *p*-hydroxymandelate as a substrate.

Membrane fractions of *E. coli* XL1 MRF'/pGEM-mdlB led to a detectible change in absorbance at 600 nm within 5 minutes with (*S*)-mandelate and with (*R*,*S*)-*p*-hydroxymandelate as a substrate. With (*R*)-mandelate, no changes in absorbance were observed.

### Example 12: Isolation of the D-p-hydroxyphenylglycine aminotransferase (HpgAT) gene

The D-*p*-hydroxyphenylglycine aminotransferase gene was isolated from *Pseudomonas putida* NCIMB 12565 (National Collection of Industrial and Marine Bacteria, Aberdeen, Scotland, UK). DNA was extracted from exponentially growing cells (OD₆₂₀ₙₘ 1.9) using the standard protocol described by Ausubel et al. (1990, *Current Protocols in Molecular Biology,* chapter 2.4.3, step 1 to 9, Whiley-Interscience, New York). The crude chromosomal DNA was treated with RNAse (20 mg/l), and subsequently with phenol/chloroform/isoamyl alcohol (25:24:1) to remove proteins.The chromosomal DNA was then partially digested with Sau3A I. The digested DNA was run on a 0.6 % agarose gel and DNA fragments between 4 and 10 kb in size were isolated.

Vector DNA was prepared by the digestion of 1 µg of pZErO-2 (Invitrogen, Groningen, The Netherlands) with *Bam*H I according to the protocol of Invitrogen.

Vector DNA and *P. putida* chromosomal DNA fragments were ligated with T4 DNA ligase. The ligation mixture was used to transform chemically competent *E. coli* Top10 cells. Transformants were plated onto LB medium with 50 mg/l kanamycin. In total 5000 colonies were obtained which formed the primary gene library. All 5000 colonies were pooled in LB medium supplemented with 50 mg/l kanamycin. After addition of glycerol to a final concentration of 15 %, the primary gene bank was stored in aliquots of 1 ml at -80°C.

Cultures of 1800 colonies were prepared in 150 µl LB medium supplemented with 50 mg/l kanamycin in microtiter plates. The cultures were cultured overnight at 28°C and harvested by centrifugation in an Eppendorf 5804 R centrifuge (Eppendorf, Hamburg, Germany). The cells were washed with 50 mM KPO₄ buffer, pH 7.0, and re-suspended in 180 µl reaction mix (100 mM potassium phosphate, pH 7.0, 15 mM α-ketoglutarate, 0.1 mM pyridoxal-phosphate and 0.5 %v/v Triton X-100).

The reaction was started by adding D-*p*-hydroxyphenylglycine to a final concentration of 5 mM. The OD₃₄₀ₙₘ in each well was monitored during 20 minutes using an Optimax microtiter plate reader (Molecular Devices, Sunnyvale, California, USA). A negative control (non transformed *E. coli* Top10) and a positive control (*P. putida* NCIMB 12565) were treated accordingly.

Of 1800 clones screened, one showed significant increase in the OD₃₄₀ₙₘ relative to the negative control due to the formation of HPGL. This clone contained the *P. putida* D-HpgAT gene on a 12 kb plasmid, pZErOTagp. Sequencing of pZErOTagp revealed the complete nucleotide sequence of the D-HpgAT gene. The sequence of this gene is listed as nucleotides 51 to 1376 of [SEQ ID: No.21, encoding the protein of SEQ.ID: No.22], as shown in annexe 3.

### Example 13: Construction of plasmid pBAD-HpgAT

The *P. putida* D-*p*-hydroxyphenylglycine aminotransferase gene was subcloned into pBAD/*Myc*-HisC using PCR. The HpgAT ORF was amplified using
5'- GTGCACGGTCTCGCATGTCTATTTATAGCGATTATGAACGTAAAAC - 3' [SEQ ID: No.23] and
5'- GTGCACGGTCTCCTCGAGTTAGCCCAGGAGGTTTTCTTCAGC - 3' [SEQ ID: No.24]
as primers (with *Bsa* I recognition and cleavage site underlined), and
chromosomal DNA of *Pseudomonas putida* NCIMB 12565 as template. Correct size (1361 bp) of the amplified fragment was confirmed by agarose gel electrophoresis.

The fragment was digested with *Bsa* I. Vector pBAD/*Myc*-HisC was digested with *Xho* I and *Nco* I. The purified digested pBAD/*Myc*-HisC vector and the digested insert DNA were ligated with T4 DNA ligase.

*E. coli* Top10 cells were transformed by electroporation with the recombinant plasmid and plated on LB medium supplemented with 100 mg/l carbenicillin.

Colony-PCR was performed and eight PCR positives as well as *E. coli* Top10/pBAD/*Myc*-HisC (as negative control) were cultured in LB medium supplemented with 100 mg/l carbenicillin with and without 0.002 % arabinose as an inducer. The cells were harvested and tested for HpgAT activity as described in Example 12. All 8 colonies showed HpgAT activity with arabinose as inducer. One of these colonies, carrying a plasmid showing the correct insert sequence (as confirmed by sequencing), and named *E. coli* pBAD-HpgAT, was deposited under the Budapest Treaty at the Deutsche Sammlung für Mikroorganismen und Zellkulturen, Braunschweig, Germany (DSMZ), on October 23^{rd}, 2000, deposit number DSM 13788.

### Example 14: Preparation of cell free extracts

*E. coli* Top10/pBAD-HpgAT was cultured in LB medium supplemented with arabinose (0.002 %) and carbenicillin (100 mg/l) overnight at 28°C. The cells were harvested by centrifugation and cell free extracts were prepared from a suspension (1 g of cells plus 7 ml of 50 mM KPO₄, pH 7.0) by sonification and subsequent centrifugation.

### Example 15.a: Activity of the D-HpgAT towards p-hydroxyphenylglyoxylate

3 ml reaction mixture containing 100 mM potassium phosphate buffer pH 7.0, 60 mM L-glutamate, 0.1 mM pyridoxal phosphate and cell free extract (0.27 mg protein, example 14) was incubated in a cuvette at 20°C. This mixture was used as blank in the spectrophotometric activity measurement at 340 nm. The reaction was started by the addition of *p*-hydroxyphenylglyoxylate (0.67 mM final concentration) into the reaction mixture. The maximum specific activity in this reaction was 110 nmole per minute per mg protein at 20°C.

### Example 15.b: Activity of the D-HpgAT towards D-PG and D-HPG

4 ml reaction mixture containing 100 mM potassium phosphate buffer pH 8.0, 15 mM α-ketoglutarate, 0.1 mM pyridoxal phosphate, and cell free extract (0.01 mg protein, example 14) was incubated at 30°C. The assay was started by the addition of the substrate D-PG (4 mM). At certain time intervals, aliquots of 1 ml were taken and transferred to 0.4 ml 1 M H₃PO₄ to stop the reaction. Samples were analyzed by HPLC (Astec Chirobiotic T 250 mm x 4.6 mm 5 µm column, Advanced Separation Technologies, Whippany, NJ, 20 µl injection volume, column temperature 22°C, 1.0 ml/min of 80 % 15 mM ammonium acetate pH 4.1 and 20 % methanol, detection at 215 nm). Within 20 min, 7% of D-PG has been converted to phenylglyoxylate, while in a similar experiment carried out with D-HPG as a substrate, 12% of D-HPG has been converted to *p-*hydroxyphenylglyoxylate.

### Example 16: pH optimum of the D-HpgAT

pH optimum of the enzyme was determined in 5 ml reaction mixtures containing 100 mM buffer of a certain pH, 6.5 mM *p-*hydroxyphenylglyoxylate, 0.05 mM pyridoxal phosphate and 100 µl cell free extract (1.35 mg protein, example 14). The reaction was started by adding 300 µmole L-glutamate (at the appropriate pH, 60 mM final concentration). After certain time intervals at 35°C, samples were taken and stopped by the addition of the same volume of 0.2 M H₃PO₄. The samples were analyzed by HPLC (Biorad HPX-87C 300 mm x 7.8 mm, 20 µl injection volume, column temperature 80°C, 1 ml/min 5 mM calcium phosphate) and detected at 210 nm.

The enzymatic activity was tested in the pH range 5 to 11. KH₂PO₄ / Na₂HPO₄ buffer (pH 5 to 8), TRIS-HCl buffer (pH 8 to 9), CHES-NaOH buffer (pH 9 to 10) and CAPS-NaOH buffer (pH 10 to 11) were applied, respectively.

The pH optimum of the enzyme was found to be between pH 8.5 and 9. Significant activity was found between pH 6 and pH 7.5. This is in contrast to Wiyakrutta and Meevootisom (1997, J. Biotechnol., 55: 193-203), who could not observe any activity below pH 7.5 of a D-HpgAT from *Pseudomonas stutzeri*.

### Example 17: Pyruvate as an amino acceptor for the D-HpgAT

The spectrophotometric assay of Example 12 (however, without Triton in the assay mixture) was applied with pyruvate instead of α-ketoglutarate as the amino acceptor. An increase in absorbance could be observed due to the formation of HPGL. Hence, *P. putida* D-HpgAT is able to use pyruvate as amino acceptor. The aminotransferase of *P. stutzeri,* described by Wiyakrutta and Meevootisom (1997, J. Biotechnol., 55: 193-203), used α-ketoglutarate as the sole amino acceptor.

### Example 18: Enantioselectivity of the D-HpgAT (I)

The spectrophotometric assay of Example 12 was used to test the activity of D-HpgAT towards L-HPG. No increase in absorption was observed when L-HPG was used as substrate. After adding D-HPG, the absorption started to increase. Hence, the present D-HpgAT is selective for D-*p*-hydroxyphenylglycine.

To test the enantioselectivity for L-glutamate, a 3 ml reaction mixture containing 100 mM potassium phosphate buffer pH 7.0, 60 mM D-glutamate and 0.1 mM pyridoxal phosphate was incubated at 20°C. This mixture was used as blank in the spectrophotometric activity measurement at 340 nm. *p*-Hydroxyphenylglyoxylate (0.5 mM final concentration) was added to the reaction mixture. After 2 minutes of incubation, the reaction was started by the addition of 20 µl cell free extract (0.27 mg protein, example 14) to the reaction mixture. No decrease in absorption could be identified when D-glutamate was used as substrate. After adding L-glutamate, the absorption started to decrease. Thus, the current D-HpgAT is selective for L-glutamate.

### Example 19: Enantioselectivity of the D-HpgAT (II)

For further proof of the strict enantioselectivity, 2 reaction mixtures (50 ml final volume) containing 100 mM potassium phosphate buffer pH 8.0, 13 mM *p*-hydroxyphenylglyoxylate, 0.05 mM pyridoxal phosphate and cell free extract (13.5 or 1.35 mg protein, example 14), were pre-incubated at 35°C for 10 minutes. The reaction was started by adding 34.8 mmole L-glutamate, pH 8.0 (final concentration 0.7 M).

Samples (1 ml) were taken after regular intervals, stopped by the addition of 2 ml of 0.2 M H₃PO₄ and analyzed by HPLC by the method of example 15. Only D-HPG (not L-HPG) was produced in the reaction.

### Experimental Part II (Examples 20-35): Construction and testing of artificial D-(H)PG biosynthesis operons

### General considerations for Examples 20-35

As expression vector for the artificial D-(H)PG biosynthesis operon, plasmid pJF119EH was chosen. This broad host range vector, constructed by Fürste, et al. (1986, Gene, 48:119-131), is suitable for protein expression in a variety of gram negative bacteria. The pJF119EH expression system uses the IPTG inducible tac promoter and carries the *lac* repressor (*lac* I^{q} gene), which keeps the expression of the cloned foreign gene in the absence of the inducer extremely low.
In all cases the different genes belonging to the D-(H)PG operon were amplified by PCR from the appropriate plasmids described in example 1, example 9, and example 13. To ensure the presence of an optimal ribosomal binding site (RBS) in front of those genes, the RBS as it is present in pBAD/*Myc*-HisC was included.

### Example 20: Construction of plasmid pJF-Sc-HmaS

The *S.* c*oelicolor p*-hydroxymandelate synthase gene was subcloned in pJF119EH using PCR. The HmaS ORF including RBS was amplified using
5' - GGGAATTCAGGAGGAATTAACCATGCCGCCgAGcGAC - 3' [SEQ ID: No.25] (with *Eco*RI restriction site underlined, start codon double underlined, and change of codon 3 and 4, indicated by non capital letters, to more frequently used ones in *E. coli*), and
5' - GAATTCCCATATTCTAGAAGGTCATCGGCCGGCCACT - 3' [SEQ ID: No.26] (with *Xba* I restriction site underlined, and stop codon double underlined) as primers, and
pBAD-Sc-HmaS (see example 1.3) plasmid DNA as template. Correct size (1120 bp) of the amplified fragment was confirmed by agarose gel electrophoresis.

The fragment and the plasmid pJF119EH were digested with *Eco*RI and *Xba* I. The two fragments were ligated and transformed in *E. coli* DH5α. Transformants were selected on LB agar plates containing 100 mg/l ampicillin. A plasmid showing the correct insert sequence (as confirmed by sequencing) was called pJF-Sc-HmaS.

### Example 21: Construction of plasmid pJF-Ao-HmaS

The *A. orientalis p*-hydroxymandelate synthase gene was subcloned in pJF119EH using PCR. The HmaS ORF including RBS was amplified using
5' - TGGGAATTCAGGAGGAATTAACCATGCAG - 3' [SEQ ID: No.27]
(with *Eco*RI restriction site underlined, and start codon double underlined), and
5' - CGGCCAGGTCTAGATACGTCATCGCCG - 3' [SEQ ID: No.28]
(with *Xba* I restriction site underlined, and stop codon double underlined) as primers, and
pBAD-Ao-HmaS (see example 1.1) plasmid DNA as template. Correct size (1115 bp) of the amplified fragment was confirmed by agarose gel electrophoresis.

The fragment and the plasmid pJF119EH were digested with *EcoR*I and *Xba* I. The two fragments were ligated and transformed in *E. coli* DH5α. Transformants were selected on LB agar plates containing 100 mg/l ampicillin. A plasmid showing the correct insert sequence (as confirmed by sequencing) was called pJF-Ao-HmaS.

### Example 22: Construction of plasmid pJF-Sc-HmaO

The *S. coelicolor p*-hydroxymandelate oxidase gene was subcloned in pJF119EH using PCR. The HmaO ORF including RBS was amplified using
5' - TGGGTCTAGAGGAGGAATTAACCATGCGcGAGCCG - 3' [SEQ ID: No.29] (with Xbal restriction site underlined, start codon double underlined and change of codon 2, indicated by non capital letters, to a more frequently used one in *E. coli*) and
5' - GAATTCCCATAGCATGCCTGGTCATCCGTGGCTCC - 3' [SEQ ID: No.30] (with *Sph* I restriction site underlined, and stop codon double underlined) as primers and
pBAD-Sc-HmaO (see example 1.4) plasmid DNA as template. Correct size (1178 bp) of the amplified fragment was confirmed by agarose gel electrophoresis.

The fragment and the plasmid pJF119EH were digested with *Xba* I and *Sph* I. The two fragments were ligated and transformed in *E. coli* DH5α. Transformants were selected on LB agar plates containing 100 mg/l ampicillin. A plasmid showing the correct insert sequence (as confirmed by sequencing) was called pJF-Sc-HmaO and used for further investigation.

### Example 23: Construction of plasmid pJF-Sc-HmaS - Sc-HmaO

In this example, the cloned Sc-HmaS and Sc-HmaO from examples 20 and 22 were combined in the expression vector pJF119EH. The Sc-HmaO gene in pJF-Sc-HmaO was excised from the expression vector by digestion with *Xba* I and *Sph* I, and this DNA fragment containing the Sc-HmaO gene was purified by gel electrophoresis. Plasmid pJF-Sc-HmaS was digested with *Xba* I and *Sph* I, ligated together with the Sc-HmaO *Xba* I / *Sph* I fragment and transformed in *E. coli* DH5α. Transformants were selected on LB agar plates containing 100 mg/l ampicillin. Different transformants were selected, plasmid minipreps prepared, and the plasmid DNA analyzed by restriction mapping. A plasmid comprising the two genes in the correct order in pJF119EH was designated pJF-Sc-HmaS - Sc-HmaO and used for further investigation.

### Example 24: Construction of plasmid pJF-Ao-HmaS - Sc-HmaO

In this example, the cloned Ao-HmaS and Sc-HmaO from examples 21 and 22 were combined in the expression vector pJF119EH, in the same way as it has been described for the construction of pJF-Sc-HmaS - Sc-HmaO in example 23. A plasmid comprising the two genes in the correct order in pJF119EH was designated pJF-Ao-HmaS - Sc-HmaO and used for further investigation.

### Example 25: Construction of plasmid pJF-HpgAT

The *P*. *putida* D-*p*-hydroxyphenylglycine aminotransferase gene was subcloned in pJF119EH using PCR. The HpgAT ORF including RBS was amplified using
5' - TTTCCCAAGCTTACAGGAGGAATTAACCATG - 3' [SEQ ID: No.31]
(with *Hin*d III restriction site underlined, start codon double underlined) and
5' - GTACCAGCTGCAAAGCTTGAGTTAGCCCAG - 3' [SEQ ID: No.32]
(with *Hin*d III restriction site underlined, and stop codon double underlined) as primers and
pBAD-HpgAT (see example 13) plasmid DNA as template. Correct size (1378 bp) of the amplified fragment was confirmed by agarose gel electrophoresis.

The fragment and the plasmid pJF119EH were digested with *Hind* III. The two fragments were ligated and transformed in *E. coli* DH5α. Transformants were selected on LB agar plates containing 100 mg/l ampicillin. Insertion of the desired fragment in same direction as the tac promoter was confirmed using restriction analysis. A plasmid showing the correct insert sequence with the exception of one silent mutation (change of codon 46 from GCG to GCA) was called pJF-HpgAT and used for further investigations.

### Example 26: Construction of plasmid pJF-Sc-HmaS - Sc-HmaO - HpgAT

In this example, the cloned HpgAT of example 25 was subcloned in plasmid pJF-Sc-HmaS - Sc-HmaO of example 23. The HpgAT gene in pJF-HpgAT was excised from the expression vector by digestion with *Hind* III, and the DNA fragment containing the HpgAT gene was purified by gel electrophoresis. Plasmid pJF-Sc-HmaS - Sc-HmaO was digested with *Hind* III. After dephosphorylation, this fragment was ligated together with the HpgAT *Hind* III fragment and transformed in *E. coli* DH5α. Transformants were selected on LB agar plates containing 100 mg/l ampicillin. Different transformants were selected, plasmid minipreps prepared, and the plasmid DNA analyzed by restriction mapping. A plasmid comprising the three genes in the correct order and direction in pJF119EH was designated pJF-Sc-HmaS - Sc HmaO - HpgAT and used for further investigation.

### Example 27: Construction of plasmid pJF-Ao-HmaS - Sc-HmaO - HpgAT

In this example, the cloned HpgAT of example 25 was subcloned in plasmid pJF-Ao-HmaS - Sc-HmaO of example 24 in the same way as described for the construction of pJF-Sc-HmaS - Sc-HmaO - HpgAT in example 26. A plasmid comprising the three genes in the correct order in pJF119EH was designated pJF-Ao-HmaS - Sc-HmaO - HpgAT and used for further investigation.

### Example 28: Construction of plasmid pJF-Sc-HmaS - mdlB - HpgAT

In this example, the cloned Sc-HmaO present in pJF-Sc-HmaS - Sc-HmaO - HpgAT (example 26) was exchanged against the *mdl*B gene of *P. putida* resulting in plasmid pJF-Sc-HmaS - mdlB - HpgAT. The *P. putida* mandelate dehydrogenase gene was amplified by PCR including the RBS sequence as it is present in plasmid pBAD/*Myc*-HisC using
5' - GGGTCTAGAGGAGGAATTAACCATGAGCCAGAATCTCTTT - 3' [SEQ ID: No.33] (with *Xba* I restriction site underlined, and start codon double underlined) and
5' - CTGCAGAACCAGCATGGTGGTCAGTACTTCACTCATGCG - 3' [SEQ ID: No.34] (with *Bst* XI restriction site underlined, and stop codon double underlined) as primers and pGEM-Bldm (see example 9) plasmid DNA as template. Correct size (1236 bp) of the amplified fragment was confirmed by agarose gel electrophoresis.

The fragment was digested with *Xba* I and BstX I. Plasmid pJF-Sc-HmaS - Sc-HmaO - HpgAT was digested with *Xba* I and *Sph* I leading to a 7692 bp fragment of the plasmid without HmaO gene. The purified 7692 bp *Xba* I / *Sph* I fragment was ligated with the mdlB ORF containing *Xba* I / *Bst*X I fragment and transformed in *E. coli* DH5α. Transformants were selected on LB agar plates containing 100 mg/l ampicillin. Different transformants were selected, plasmid minipreps prepared, and the plasmid DNA analyzed by restriction mapping. A plasmid comprising the three genes in the correct order in pJF119EH was designated pJF-Sc-HmaS - mdlB - HpgAT and used for further investigation.

### Example 29: Construction of plasmid pJF-Ao-HmaS - mdIB - HpgAT

In this example, the cloned Sc-HmaO present in pJF-Ao-HmaS - Sc-HmaO - HpgAT (example 27) was exchanged against the *mdl*B gene of *P. putida* resulting in plasmid pJF-Ao-HmaS - mdIB - HpgAT, in the same way as described for the construction of pJF-Sc-HmaS - mdlB - HpgAT in example 28.

A plasmid comprising the three genes in the correct order in pJF119EH was designated pJF-Ao-HmaS - mdlB - HpgAT and used for further investigation.

### Example 30: Expression of artificial D-(H)PG cluster on plasmids pJF-Sc-HmaS-Sc-HmaO-HpgAT, pJF-Ao-HmaS-Sc-HmaO-HpgAT, pJF-Sc-HmaS-mdIB-HpgAT, and pJF-Ao-HmaS-mdIB-HpgAT

Single colonies of the *E. coli* DH5α strains harboring the plasmids pJF-Sc-HmaS-Sc-HmaO-HpgAT, pJF-Ao-HmaS-Sc-HmaO-HpgAT, pJF-Sc-HmaS-mdIB-HpgAT, or pJF-Ao-HmaS-mdIB-HpgAT were used to inoculate 10 ml of LB medium containing Ampicillin (100 µg/ml) and incubated at 30°C for 16 hours.
1 ml of these cultures were subsequently used to inoculate 50 ml of the same medium. Cells were grown at 30°C with 180 rpm. At OD₆₂₀ₙₘ 0.8, the cells were induced by the addition of 0.1 mM IPTG. After 4 hours, the cells were harvested and washed with 100 mM potassium phosphate buffer, pH 7.5. Aliquots of washed cells were frozen at -20°C for later use. As a control *E. coli* DH5α harboring plasmid pJF119EH was treated accordingly.

Crude extracts were prepared with B-PER^{™} (in phosphate buffer) (Pierce, Rockford, Illinois, USA) immediately before use.

### Example 31: In vitro production of D-HPG from p-hydroxyphenylpyruvate

The assay mixture of 3 ml contained 200 mM potassium phosphate buffer pH 8.0, 5 mM *p*-hydroxyphenylpyruvate (HPP), 10% ethanol (50 mM *p*-hydroxyphenylpyruvate stock solution in 96 % ethanol was used), 44 mM ascorbate, 40 mM L-glutamate, 40 mM NAD⁺, 0.1 mM pyridoxal phosphate, and cell free extract of example 30 leading to a final concentration of 0.6 mg/ml soluble protein.

The assay was started by the addition of HPP and stopped after 65 h at 30°C by the addition of 0.1 ml 1 N HCl to an aliquot of 0.5 ml of the reaction system. The samples were analyzed by HPLC as described in example 3.
The amounts of *p*-hydroxymandelate (HMA), *p*-hydroxyphenylglyoxylate (HPGL), and D-HPG produced in mg/l are summarized in Table1.

**Table 1:**

| Plasmid | HMA | HPGL | D-HPG |
|---|---|---|---|
| pJF-Sc-HmaS - Sc-HmaO - HpgAT | 17 | 67 | 9 |
| pJF-Ao-HmaS - Sc-HmaO - HpgAT | 0 | 166 | 11 |
| pJF-Sc-HmaS - mdlB - HpgAT | 37 | 73 | 24 |
| pJF-Ao-HmaS - mdlB - HpgAT | 0 | 97 | 26 |
| pJF119EH | 0 | 0 | 0 |

As can be seen, none of these compounds (HMA, HPGL and D-HPG) was detected in control experiments with cell free extracts derived from *E. coli* DH5α/pJF119EH.

### Example 32: In vitro production of D-PG from phenylpyruvate

The assay mixture of 3 ml contained 200 mM potassium phosphate buffer pH 8.0, 5 mM phenylpyruvate (PP), 44 mM ascorbate, 40 mM L-glutamate, 40 mM NAD⁺, 0.1 mM pyridoxal phosphate, and cell free extract of example 30 leading to a final concentration of 0.3 mg/ml soluble protein.

The assay was started by the addition of PP and stopped after 39 h at 30°C by the addition of 0.1 ml 1 N HCl to an aliquot of 0.5 ml of the reaction system. The samples were analyzed by HPLC as described in example 3.

Within 39 h 32 mg/l D-PG were produced with cell free extracts derived from *E. coli* DH5α/ pJF-Ao-HmaS - Sc-HmaO - HpgAT, while 27 mg/l D-PG were produced with cell free extracts derived from *E. coli* DH5α/ pJF-Ao-HmaS - mdlB - HpgAT. No D-PG was detected in control experiments with cell free extracts derived from *E. coli* DH5α/ pJF119EH.

### Example 33: Construction of plasmid pCR-Bl-tyrA

The *tyr*A ORF encoding the *E. coli* chorismate mutase - prephenate dehydrogenase including the original RBS of *E. coli* (nucleotides 4740 - 5877 of accession AE000346) was amplified using
5' - GCGTGGAAGCTTAAGAGGTTTATTATGGTTGCTGAA - 3' [SEQ ID: No.35] (with *Hind* III restriction site underlined, and start codon double underlined) and
5' - GTGCACGGTCTCGAGCTGAATTCTTACTGGCGATTGTCAT - 3' [SEQ ID: No.36] (with *Bsa* I recognition and cleavage site underlined, and stop codon double underlined) as primers, and
chromosomal DNA of the wild type *E. coli* strain LJ110 (Zeppenfeld et al 2000) as a template. Correct size (1169 bp) of the amplified fragment was confirmed by agarose gel electrophoresis.

The amplified fragment was directly inserted, according to the instruction of the supplier, into the vector pCR®-Blunt II-TOPO® (Invitrogen, Groningen, The Netherlands) and transformed in chemically competent *E. coli* Top10 (Invitrogen, Groningen, The Netherlands). The transformants were selected on LB agar plates containing 50 mg/l kanamycin. A plasmid showing the correct insert sequence (as confirmed by sequencing) was called pCR-Bl-tyrA and used for further investigations.

### Example 34: Construction of plasmid pJF- Sc-HmaS - mdlB - HpgAT - tyrA

As a host strain for D-HPG production *E. coli* KB532 (Δ(*phe*A-*tyr*A), Δ*tyr*R, *aro*F^{fbr}, *thi*A, *hsd*R17, *end*A1, *sup*E44) was chosen. KB532 is a L-tyrosine and L-phenylalanine auxotrophic strain. It lacks the genes for *tyr*A (chorismate mutase/prephenate dehydrogenase), *phe*A (chorismate mutase/prephenate dehydratase), and for the global regulator *tyr*R, and carries *aro*F^{fbr} encoding a feed back (L-tyrosine) resistant DAHP synthase. Overexpressing the *tyr*A gene in KB532 leads to a tyrosine producing *E. coli* strain, and therewith also to a strain delivering higher amounts of *p*-hydroxyphenylpyruvate.

To enable the overexpression of *tyr*A together with the artificial D-(H)PG operon, the cloned *tyr*A gene of example 33 was subcloned in plasmid pJF-Sc-HmaS - mdlB - HpgAT of example 28. The *tyr*A gene together with its RBS in pCR-BI-tyrA was excised from the expression vector by digestion with *Hind* III and *Bsa* I, and the DNA fragment containing the *tyr*A ORF was purified by gel electrophoresis.

Partial digestion of plasmid pJF- Sc-HmaS - mdlB - HpgAT with *Hin*d III was performed to obtain fragments of the linearised plasmid, because pJF- Sc-HmaS - mdlB - HpgAT contains three *Hin*d III sites. Correct size of the restriction fragments was confirmed by agarose gel electrophoresis and the fragments of 8912 bp corresponding with the linearised plasmid were purified from the gel. After treatment with alkaline phosphatase to prevent recircularisation of the plasmid, these 8912 bp fragments were ligated with the tyrA *Hind* III / *Bsa* I fragment.

Recombinant plasmids were selected by complementation by transformation in the *tyr*A- deficient *E. coli* strain, KB532 (see above) and growth on minimal medium supplemented with 50 mg/l L-phenylalanine and 0.01 mM IPTG. Different transformants were selected, plasmid minipreps prepared, and the plasmid DNA analyzed by restriction mapping. A plasmid comprising the four genes in the correct order and direction in pJF119EH was designated pJF-Sc-HmaS - mdlB - HpgAT - tyrA, the appropriate strain was called KB532/pJF-Sc-HmaS - mdlB - HpgAT - tyrA and used for further investigation.

### Example 35: Fermentative production of D-HPG

The D-HPG production of *E. coli* KB532/pJF- Sc-HmaS - mdlB - HpgAT - tyrA from glucose was investigated in mineral medium. This mineral medium consisted of Na citrate·3H₂O (1.0 g·l⁻¹), MgSO₄·7H₂O (0.3 g·l⁻¹), KH₂PO₄ (3.0 g·l⁻¹), K₂HPO₄ (12.0 g·l⁻¹), NaCl (0.1 g·l⁻¹), (NH₄)₂SO₄ (5.0 g·l⁻¹), CaCl₂·2H₂O (15.0 mg·l⁻¹), FeSO₄·7H₂O (75.0 mg·l⁻¹), thiamine·HCl (vitamin B1) (5.0 mg·l⁻¹), and L-phenylalanine (0.05 g·l⁻¹). Additional minerals were added in the form of a trace element solution (1 ml·l⁻¹), which trace element solution was composed of Al₂(SO₄)₃·18H₂O (2.0 g·l⁻¹), CoCl₂·6H₂O (0.7 g·l⁻¹), CuSO₄·5H₂O (2.5 g·l⁻¹). H₃BO₃ (0.5 g·l⁻¹), MnCl₂·4H₂O (20.0 g·l⁻¹) Na₂MoO₄·2H₂O (3.0 g·l⁻¹), NiSO₄·6H₂O (2.0 g·l⁻¹), ZnSO₄·7H₂O (15.0 g·l⁻¹). A stock solution of Glucose (30 g·l⁻¹) was autoclaved separately and added to the sterilized medium to a final concentration of 4 g·l⁻¹.

A single colony of the *E. coli* KB532/pJF- Sc-HmaS - mdIB - HpgAT - tyrA was used to inoculate 10 ml of minimal medium containing ampicillin (100 µg/ml) and incubated at 30°C for 16 hours. 4 ml of this culture was subsequently used to inoculate 50 ml of the same medium and incubated at 33°C and 180 rpm for 24 h. After 12 h at an OD₆₂₀ₙₘ of ~ 0.6, the cells were induced by adding 0.1 mM IPTG. A sample of the culture supernatant was adjusted to pH 5.8, lyophilized, and redissolved in D₂O. 600 MHz ¹H-NMR at 323 K showed the expected resonance spectrum and spiking with a small amount of HPG confirmed the presence of HPG. Redundant evidence was obtained from a 2-D COSY experiment. The amount present was determined to be 5 mg/l. (Besides HPG, 80 mg/l of its precursor *p*-hydroxyphenylglyoxylate was present, but no *p-*hydroxymandelate.)

### Annexe 1.1

### [SEQ ID: No.9]

[CDS for p-hydroxymandelate synthase from *Nocardia uniformis* subsp. *tsuyamanensis* ATCC 21806]

aaaccgtcta taaatgctgc cggagggcga tcccacggct ggaggttcgt g atg gcg 57
Met Ala
1

gca cag gca ggc agc gtg ttc gac ggc atg acg ctc gac cac acc gtg 105
Ala Gln Ala Gly Ser Val Phe Asp Gly Met Thr Leu Asp His Thr Val
        5                   10                  15

ttc tac gtc ggc gac gcg gac cgc gcg gcg ggc gag ctc acc gac aag 153
Phe Tyr Val Gly Asp Ala Asp Arg Ala Ala Gly Glu Leu Thr Asp Lys
    20                  25                  30

tac ggg ctg gtg gtg ctc ggc acg tcc gag acc tcg tcg gtg cgc tcg 201
Tyr Gly Leu Val Val Leu Gly Thr Ser Glu Thr Ser Ser Val Arg Ser
35                  40                  45                  50

gtc gcg gtg ggc ggc ggc tcc atc cgg ctg gtg ttc tcc cag gcc atc 249
Val Ala Val Gly Gly Gly Ser Ile Arg Leu Val Phe Ser Gln Ala Ile
                55                  60                  65

gcc gac gac acc ccg gct gcc gcg tac gtg aag gtg cac ggc gac ggc 297
Ala Asp Asp Thr Pro Ala Ala Ala Tyr Val Lys Val His Gly Asp Gly
            70                  75                  80

gtg gcc gac ctc gcg ctg ggc gtc gcc gac gcc cgc gcc gcg ttc gcc 345
Val Ala Asp Leu Ala Leu Gly Val Ala Asp Ala Arg Ala Ala Phe Ala
        85                  90                  95

gag gcg gtg cgg cgg ggc gcg cgg ccg gtg gcc gag ccc acc gag gcc 393
Glu Ala Val Arg Arg Gly Ala Arg Pro Val Ala Glu Pro Thr Glu Ala
    100                 105                 110

gac ggc gcg gtg ctc gcc acg atc atg ggc ttc ggc gac gtg gtg cac 441
Asp Gly Ala Val Leu Ala Thr Ile Met Gly Phe Gly Asp Val Val His
115                 120                 125                 130

acc ttc gtc cag cgc ccc ggc ggc gcg ccc ggc gag gac ccg gag agc 489
Thr Phe Val Gln Arg Pro Gly Gly Ala Pro Gly Glu Asp Pro Glu Ser
                135                 140                 145

gcg ggc ggc ctg cgc gtg ctg gac cac ttc gcg gtc tgc ctc gag gcg 537
Ala Gly Gly Leu Arg Val Leu Asp His Phe Ala Val Cys Leu Glu Ala
            150                 155                 160

ggc ggg ctg gag ccg acc gtg gcg ttc tac cag gag gtg ctg gac ttc 585
Gly Gly Leu Glu Pro Thr Val Ala Phe Tyr Gln Glu Val Leu Asp Phe
        165                 170                 175

cgg gtg gtc ttc gag gag aag atc gtc gtc ggg gcg cag gcg atg aac 633
Arg Val Val Phe Glu Glu Lys Ile Val Val Gly Ala Gln Ala Met Asn
    180                 185                 190

tcc aag gtc gtg cag agc acg tcc ggc gcg gtc acg ctg acc ctc atc 681
Ser Lys Val Val Gln Ser Thr Ser Gly Ala Val Thr Leu Thr Leu Ile
195                 200                 205                 210

gaa ccg gac acc tcg cgc aag ccc ggt cag atc gac gac ttc atc aag 729
Glu Pro Asp Thr Ser Arg Lys Pro Gly Gln Ile Asp Asp Phe Ile Lys
                215                 220                 225

aac cac ggt ggc gcg ggc gtg cag cac atc gcc ttc gcc acc gac ggc 777
Asn His Gly Gly Ala Gly Val Gln His Ile Ala Phe Ala Thr Asp Gly
            230                 235                 240

atc gtg gac gcg gtg cgc cgg ttg cgc gag cgg ggc gtg gag ctg ctc 825
Ile Val Asp Ala Val Arg Arg Leu Arg Glu Arg Gly Val Glu Leu Leu
        245                 250                 255

acc acg ccc gcc gcc tac tac gac ctg ctg gcg gac cgc ctc gga ccg 873
Thr Thr Pro Ala Ala Tyr Tyr Asp Leu Leu Ala Asp Arg Leu Gly Pro
    260                 265                 270

acc cgg tac tcc acg gcc gag ctg gcc gag ctg aac ctg ctg gtc gac 921

Thr Arg Tyr Ser Thr Ala Glu Leu Ala Glu Leu Asn Leu Leu Val Asp
275                 280                 285                 290

gag gac cag gac ggc aag ctg tac cag atc ttc gcc cgg tcc acc cac 969
Glu Asp Gln Asp Gly Lys Leu Tyr Gln Ile Phe Ala Arg Ser Thr His
                295                 300                 305

ccc agg ggc acg ttc ttc ttc gag atc atc gag cgc gcg ggc gcg cac 1017
Pro Arg Gly Thr Phe Phe Phe Glu Ile Ile Glu Arg Ala Gly Ala His
            310                 315                 320

acc ttc ggc agc ggc aac atc aag gcc ctc tac gag gcc gtc gag gcc 1065
Thr Phe Gly Ser Gly Asn Ile Lys Ala Leu Tyr Glu Ala Val Glu Ala
        325                 330                 335

gag cgc cac cgg acc gag cgc tgagcgcggg gcacggcgat ggacccgctg 1116
Glu Arg His Arg Thr Glu Arg
    340                 345

cgcgccggtg acctgcacgg ctcg 1140

### Annexe 1.2

### [SEQ ID: No.10]

[p-hydroxymandelate synthase from *Nocardia uniformis* subsp. *tsuyamanensis* ATCC 21806]

Met Ala Ala Gln Ala Gly Ser Val Phe Asp Gly Met Thr Leu Asp His
1               5                   10                  15

Thr Val Phe Tyr Val Gly Asp Ala Asp Arg Ala Ala Gly Glu Leu Thr
            20                  25                  30

Asp Lys Tyr Gly Leu Val Val Leu Gly Thr Ser Glu Thr Ser Ser Val
        35                  40                  45

Arg Ser Val Ala Val Gly Gly Gly Ser Ile Arg Leu Val Phe Ser Gln
    50                  55                  60

Ala Ile Ala Asp Asp Thr Pro Ala Ala Ala Tyr Val Lys Val His Gly
65                  70                  75                  80

Asp Gly Val Ala Asp Leu Ala Leu Gly Val Ala Asp Ala Arg Ala Ala
                85                  90                  95

Phe Ala Glu Ala Val Arg Arg Gly Ala Arg Pro Val Ala Glu Pro Thr
            100                 105                 110

Glu Ala Asp Gly Ala Val Leu Ala Thr Ile Met Gly Phe Gly Asp Val
        115                 120                 125

Val His Thr Phe Val Gln Arg Pro Gly Gly Ala Pro Gly Glu Asp Pro
    130                 135                 140

Glu Ser Ala Gly Gly Leu Arg Val Leu Asp His Phe Ala Val Cys Leu
145                 150                 155                 160

Glu Ala Gly Gly Leu Glu Pro Thr Val Ala Phe Tyr Gln Glu Val Leu
                165                 170                 175

Asp Phe Arg Val Val Phe Glu Glu Lys Ile Val Val Gly Ala Gln Ala
            180                 185                 190

Met Asn Ser Lys Val Val Gln Ser Thr Ser Gly Ala Val Thr Leu Thr
        195                 200                 205

Leu Ile Glu Pro Asp Thr Ser Arg Lys Pro Gly Gln Ile Asp Asp Phe
    210                 215                 220

Ile Lys Asn His Gly Gly Ala Gly Val Gln His Ile Ala Phe Ala Thr
225                 230                 235                 240

Asp Gly Ile Val Asp Ala Val Arg Arg Leu Arg Glu Arg Gly Val Glu
                245                 250                 255

Leu Leu Thr Thr Pro Ala Ala Tyr Tyr Asp Leu Leu Ala Asp Arg Leu
            260                 265                 270

Gly Pro Thr Arg Tyr Ser Thr Ala Glu Leu Ala Glu Leu Asn Leu Leu
        275                 280                 285

Val Asp Glu Asp Gln Asp Gly Lys Leu Tyr Gln Ile Phe Ala Arg Ser
    290                 295                 300

Thr His Pro Arg Gly Thr Phe Phe Phe Glu Ile Ile Glu Arg Ala Gly
305                 310                 315                 320

Ala His Thr Phe Gly Ser Gly Asn Ile Lys Ala Leu Tyr Glu Ala Val
                325                 330                 335

Glu Ala Glu Arg His Arg Thr Glu Arg
            340                 345

### Annexe 2.1

### [SEQ ID: No.15]

[CDS for p-hydroxymandelate oxidase from *Nocardia uniformis* subsp. *tsuyamanensis* ATCC 21806]

ccccgcgcgt ggggaggcgg cgtgcccgac ggacaggagg caacgagcc atg ggc gtc 58
Met Gly Val
1

cgc aac tcc gca ggg ggc ggc gcg gaa gac ccc gag gac ctg gct gag 106
Arg Asn Ser Ala Gly Gly Gly Ala Glu Asp Pro Glu Asp Leu Ala Glu
    5                   10                  15

gtg gaa cgg gcc gcc gcc gcc cgg ctg ccg ggg gac gtg cgc gac ttc 154
Val Glu Arg Ala Ala Ala Ala Arg Leu Pro Gly Asp Val Arg Asp Phe
20                  25                  30                  35

atc gcg ggc ggc agc ggc gac gag gtg acg ctg gcc gcc aac cgc gcg 202
Ile Ala Gly Gly Ser Gly Asp Glu Val Thr Leu Ala Ala Asn Arg Ala
                40                  45                  50

gcg ctc gac gac gtg gcc ctg ctc ccc agg gtg ctc gcg ggt gtc cag 250
Ala Leu Asp Asp Val Ala Leu Leu Pro Arg Val Leu Ala Gly Val Gln
            55                  60                  65

gcg gcc gac acg agc acc tcg ctc gtg ggc acg gcg gcc acg ctg ccc 298
Ala Ala Asp Thr Ser Thr Ser Leu Val Gly Thr Ala Ala Thr Leu Pro
        70                  75                  80

gtc gcc gtg gcg ccg atg ggc tac cag tgc ctc gtc cac ccc gac ggc 346
Val Ala Val Ala Pro Met Gly Tyr Gln Cys Leu Val His Pro Asp Gly
    85                  90                  95

gag gtc gcg gct gcc gcc gcg gcg ggc gcc gcc ggc gtc ccc ttc acc 394
Glu Val Ala Ala Ala Ala Ala Ala Gly Ala Ala Gly Val Pro Phe Thr
100                 105                 110                 115

gtc ggc acg ctg agc agc cgg tcg gtc gag gag atc gcc gag acc ggc 442
Val Gly Thr Leu Ser Ser Arg Ser Val Glu Glu Ile Ala Glu Thr Gly
                120                 125                 130

gcg tcg ctg tgg ttc cag ctc tac tgg ctg cgc gat cgc ggc ctg gtc 490
Ala Ser Leu Trp Phe Gln Leu Tyr Trp Leu Arg Asp Arg Gly Leu Val
            135                 140                 145

gcc gaa ctc gtg gcg cgg gcc gag gcg gcg ggc tgc cgg gcg ctg gtg 538
Ala Glu Leu Val Ala Arg Ala Glu Ala Ala Gly Cys Arg Ala Leu Val
        150                 155                 160

atc acc gtg gac gtg ccg gtg atg ggc cgc agg ctc cgg gac gtg cgc 586
Ile Thr Val Asp Val Pro Val Met Gly Arg Arg Leu Arg Asp Val Arg
    165                 170                 175

aac ggg atc acc ctg ccc cgg acc gtc cgg gcc gtc cac ctc gcc gac 634
Asn Gly Ile Thr Leu Pro Arg Thr Val Arg Ala Val His Leu Ala Asp
180                 185                 190                 195

ggc ccg tca tcc gcg cac gag ccg cgc cag gtc ggc tcc ggc gtc gcc 682
Gly Pro Ser Ser Ala His Glu Pro Arg Gln Val Gly Ser Gly Val Ala
                200                 205                 210

cag cac acg agc gcg gtc ttc gac ccc gcg ttc ggg tgg cgc gac ctg 730
Gln His Thr Ser Ala Val Phe Asp Pro Ala Phe Gly Trp Arg Asp Leu
            215                 220                 225

gag tgg ctg cgg gcg cgc acc agg ctc ccc ctg gtg gtc aag ggc gtg 778
Glu Trp Leu Arg Ala Arg Thr Arg Leu Pro Leu Val Val Lys Gly Val
        230                 235                 240

ctc gac ccg cgc gac gcc acc agg tgc gtc gag ctg ggc gcc tcg gcg 826
Leu Asp Pro Arg Asp Ala Thr Arg Cys Val Glu Leu Gly Ala Ser Ala
    245                 250                 255

gtg gtg gtg tcc aac cac ggc ggg cgg cag ctc gac ggc gcg gcg ccc 874
Val Val Val Ser Asn His Gly Gly Arg Gln Leu Asp Gly Ala Ala Pro
260                 265                 270                 275

agc gcg gtg gcc ctg ccg cgc gtc gtc gac gcg gtg gcg ggc gcg gcc 922

Ser Ala Val Ala Leu Pro Arg Val Val Asp Ala Val Ala Gly Ala Ala
                280                 285                 290

gag gtg ctg ttc gac agc ggc gtc cgc ggc ggc gtc gac gtg ctg cgc 970
Glu Val Leu Phe Asp Ser Gly Val Arg Gly Gly Val Asp Val Leu Arg
            295                 300                 305

gcc ctc gcg ctc ggc gcg acc ggc gtg ctg ctc ggc cgc cca atc ctg 1018
Ala Leu Ala Leu Gly Ala Thr Gly Val Leu Leu Gly Arg Pro Ile Leu
        310                 315                 320

tgg ggg ctc gcg gtc ggc ggc gag cgc ggc gcg gcg cgg gtg ctg gaa 1066
Trp Gly Leu Ala Val Gly Gly Glu Arg Gly Ala Ala Arg Val Leu Glu
    325                 330                 335

ctg ctg cgc acc gag ttc gcg cag gcc ctg ctg ctc gcc ggg tgc gcc 1114
Leu Leu Arg Thr Glu Phe Ala Gln Ala Leu Leu Leu Ala Gly Cys Ala
340                 345                 350                 355

gac gtc gac gcg gcc agg gga ctc gcg acc gcg cag gcc gcg ccg acc 1162
Asp Val Asp Ala Ala Arg Gly Leu Ala Thr Ala Gln Ala Ala Pro Thr
                360                 365                 370

cgg cga ggt gcg ccc tgaccgcctc ggggaccgcg atcgggccg gggcgcgggg 1217
Arg Arg Gly Ala Pro
            375

tcaggggaag 1227

### Annexe 2.2

### [SEQ ID: No.16]

[p-hydroxymandelate oxidase from *Nocardia uniformis* subsp. *tsuyamanensis* ATCC 21806]

Met Gly Val Arg Asn Ser Ala Gly Gly Gly Ala Glu Asp Pro Glu Asp
1               5                   10                  15

Leu Ala Glu Val Glu Arg Ala Ala Ala Ala Arg Leu Pro Gly Asp Val
            20                  25                  30

Arg Asp Phe Ile Ala Gly Gly Ser Gly Asp Glu Val Thr Leu Ala Ala
        35                  40                  45

Asn Arg Ala Ala Leu Asp Asp Val Ala Leu Leu Pro Arg Val Leu Ala
    50                  55                  60

Gly Val Gln Ala Ala Asp Thr Ser Thr Ser Leu Val Gly Thr Ala Ala
65                  70                  75                  80

Thr Leu Pro Val Ala Val Ala Pro Met Gly Tyr Gln Cys Leu Val His
                85                  90                  95

Pro Asp Gly Glu Val Ala Ala Ala Ala Ala Ala Gly Ala Ala Gly Val
            100                 105                 110

Pro Phe Thr Val Gly Thr Leu Ser Ser Arg Ser Val Glu Glu Ile Ala
        115                 120                 125

Glu Thr Gly Ala Ser Leu Trp Phe Gln Leu Tyr Trp Leu Arg Asp Arg
    130                 135                 140

Gly Leu Val Ala Glu Leu Val Ala Arg Ala Glu Ala Ala Gly Cys Arg
145                 150                 155                 160

Ala Leu Val Ile Thr Val Asp Val Pro Val Met Gly Arg Arg Leu Arg
                165                 170                 175

Asp Val Arg Asn Gly Ile Thr Leu Pro Arg Thr Val Arg Ala Val His
            180                 185                 190

Leu Ala Asp Gly Pro Ser Ser Ala His Glu Pro Arg Gln Val Gly Ser
        195                 200                 205

Gly Val Ala Gln His Thr Ser Ala Val Phe Asp Pro Ala Phe Gly Trp
    210                 215                 220

Arg Asp Leu Glu Trp Leu Arg Ala Arg Thr Arg Leu Pro Leu Val Val
225                 230                 235                 240

Lys Gly Val Leu Asp Pro Arg Asp Ala Thr Arg Cys Val Glu Leu Gly
                245                 250                 255

Ala Ser Ala Val Val Val Ser Asn His Gly Gly Arg Gln Leu Asp Gly
            260                 265                 270

Ala Ala Pro Ser Ala Val Ala Leu Pro Arg Val Val Asp Ala Val Ala
        275                 280                 285

Gly Ala Ala Glu Val Leu Phe Asp Ser Gly Val Arg Gly Gly Val Asp
    290                 295                 300

Val Leu Arg Ala Leu Ala Leu Gly Ala Thr Gly Val Leu Leu Gly Arg
305                 310                 315                 320

Pro Ile Leu Trp Gly Leu Ala Val Gly Gly Glu Arg Gly Ala Ala Arg
                325                 330                 335

Val Leu Glu Leu Leu Arg Thr Glu Phe Ala Gln Ala Leu Leu Leu Ala
            340                 345                 350

Gly Cys Ala Asp Val Asp Ala Ala Arg Gly Leu Ala Thr Ala Gln Ala
        355                 360                 365

Ala Pro Thr Arg Arg Gly Ala Pro
    370                 375

### Annexe 3.1

### [SEQ ID: No.21]

[CDS for D-p-hydroxyphenylglycine aminotransferase from *Pseudomonas putida* NCIMB 12565]

tcagttaact gccatgtaat ctcaaaaacc agtccaatag aggcatcagc atg tct 56
Met Ser
1

att tat agc gat tat gaa cgt aaa acg gtg ggc tcc gcg cat tgg gcg 104
Ile Tyr Ser Asp Tyr Glu Arg Lys Thr Val Gly Ser Ala His Trp Ala
        5                   10                  15

gat cgg gca cga gct gta atg cca gat ggc gta acg gct gat aca cgt 152
Asp Arg Ala Arg Ala Val Met Pro Asp Gly Val Thr Ala Asp Thr Arg
    20                  25                  30

gtt ttt gat ccg cat ggg ctg tac atc tgt gac gcg caa ggt gtt cac 200
Val Phe Asp Pro His Gly Leu Tyr Ile Cys Asp Ala Gln Gly Val His
35                  40                  45                  50

aag acc gat gtt gat ggc aac gta tac ctg gat ttt ttt ggt ggg cat 248
Lys Thr Asp Val Asp Gly Asn Val Tyr Leu Asp Phe Phe Gly Gly His
                55                  60                  65

ggc gct tta gtg ctt ggt cat gga cat cca cgt att aac aag gca atc 296
Gly Ala Leu Val Leu Gly His Gly His Pro Arg Ile Asn Lys Ala Ile
            70                  75                  80

tct gca gcg ctg acg cat ggc gtc caa tac ggt gca agc cat ccg ctt 344
Ser Ala Ala Leu Thr His Gly Val Gln Tyr Gly Ala Ser His Pro Leu
        85                  90                  95

gaa gtg cga tgg gcc gag cgc ttg gtc aac gcc ttc cct tcg atg cat 392
Glu Val Arg Trp Ala Glu Arg Leu Val Asn Ala Phe Pro Ser Met His
    100                 105                 110

aaa gtg cgc ttt gcc ggc agc ggc act gag gcg act tta ttg gca ctg 440
Lys Val Arg Phe Ala Gly Ser Gly Thr Glu Ala Thr Leu Leu Ala Leu
115                 120                 125                 130

cga atg gcg cgg gcg ttt act ggc agg tcg aaa ata cta cgt att gcc 488
Arg Met Ala Arg Ala Phe Thr Gly Arg Ser Lys Ile Leu Arg Ile Ala
                135                 140                 145

acc cac tat cat ggc tgg cat gac ttc tcc gcc tct ggt tac aac agt 536
Thr His Tyr His Gly Trp His Asp Phe Ser Ala Ser Gly Tyr Asn Ser
            150                 155                 160

cac ttc gac ggc cag cca gca ccg ggc gtc ctt gct gaa att gca cag 584
His Phe Asp Gly Gln Pro Ala Pro Gly Val Leu Ala Glu Ile Ala Gln
        165                 170                 175

agc aca cta ctg gtt cgc cca gat gac ttc gaa gga tta cgc gca cta 632
Ser Thr Leu Leu Val Arg Pro Asp Asp Phe Glu Gly Leu Arg Ala Leu
    180                 185                 190

ttc gct caa tac ggc ggt gag att gcg acc att att gcg gag cca gtc 680
Phe Ala Gln Tyr Gly Gly Glu Ile Ala Thr Ile Ile Ala Glu Pro Val
195                 200                 205                 210

ggc tct cat ttc ggt atc act ccg gtc agc gac gat ttc ttg cta gaa 728
Gly Ser His Phe Gly Ile Thr Pro Val Ser Asp Asp Phe Leu Leu Glu
                215                 220                 225

ggc gcc gcg ctg gct cga aag cac ggt gca ata ttc att ctt gac gaa 776
Gly Ala Ala Leu Ala Arg Lys His Gly Ala Ile Phe Ile Leu Asp Glu
            230                 235                 240

gtg ata acc ggt ttt cgc gtg ggt aac cac ggt atg cag ggg ctt ctc 824
Val Ile Thr Gly Phe Arg Val Gly Asn His Gly Met Gln Gly Leu Leu
        245                 250                 255

gac att gca ccg gat ctt acg tgc ttg gcc aag gcc agt gct gga ggt 872
Asp Ile Ala Pro Asp Leu Thr Cys Leu Ala Lys Ala Ser Ala Gly Gly
    260                 265                 270

ctt cct gga ggc gta gtc gga gga agg gcg gat gtc atg gcg gta ctg 920
Leu Pro Gly Gly Val Val Gly Gly Arg Ala Asp Val Met Ala Val Leu
275                 280                 285                 290

gat cgc ggt tca gac cgt aag gtt ctg cat caa ggc acc ttc acc gga 968
Asp Arg Gly Ser Asp Arg Lys Val Leu His Gln Gly Thr Phe Thr Gly
                295                 300                 305

aac cca att acg gcg gcg gca gca atc gcg gcg atc gat acc att atc 1016
Asn Pro Ile Thr Ala Ala Ala Ala Ile Ala Ala Ile Asp Thr Ile Ile
            310                 315                 320

gaa gat gac gtg tgc aca cac atc aat gcc ctc ggt caa tac gcc cgg 1064
Glu Asp Asp Val Cys Thr His Ile Asn Ala Leu Gly Gln Tyr Ala Arg
        325                 330                 335

gac tcg atg aac gaa ctc ttc gct cgt aag cat ttg gac tgg ttg gct 1112
Asp Ser Met Asn Glu Leu Phe Ala Arg Lys His Leu Asp Trp Leu Ala
    340                 345                 350

tat ggc cgc ttt tcc ggc ttc cac ctg atg ccg gga tta tct ccg ctg 1160
Tyr Gly Arg Phe Ser Gly Phe His Leu Met Pro Gly Leu Ser Pro Leu
355                 360                 365                 370

acc act gac acg ggt gtg atc act cgc gga gaa gtt gca cgg ccc gca 1208
Thr Thr Asp Thr Gly Val Ile Thr Arg Gly Glu Val Ala Arg Pro Ala
                375                 380                 385

gta aaa atg att gcc gct atg cgc atg gca tta atc ctt gag ggc atc 1256
Val Lys Met Ile Ala Ala Met Arg Met Ala Leu Ile Leu Glu Gly Ile
            390                 395                 400

gat att ggt ggc cgt gga tct gtg ttt ctc tct gcg cag cat gat cgc 1304
Asp Ile Gly Gly Arg Gly Ser Val Phe Leu Ser Ala Gln His Asp Arg
        405                 410                 415

gga cat gtc gac caa ctg gtg tcg acc ttt gat tcc att ctt ggc cgc 1352
Gly His Val Asp Gln Leu Val Ser Thr Phe Asp Ser Ile Leu Gly Arg
    420                 425                 430

ctg gct gaa gaa aac ctc ctg ggc taagccctta cacatctgtc tggaatatga 1406
Leu Ala Glu Glu Asn Leu Leu Gly
435                 440

atcatgaaaa ctagcctgaa a 1427

### Annexe 3.2

### [SEQ ID: No.22]

[D-p-hydroxyphenylglycine aminotransferase from *Pseudomonas putida* NCIMB 12565]

Met Ser Ile Tyr Ser Asp Tyr Glu Arg Lys Thr Val Gly Ser Ala His
1               5                   10                  15

Trp Ala Asp Arg Ala Arg Ala Val Met Pro Asp Gly Val Thr Ala Asp
            20                  25                  30

Thr Arg Val Phe Asp Pro His Gly Leu Tyr Ile Cys Asp Ala Gln Gly
        35                  40                  45

Val His Lys Thr Asp Val Asp Gly Asn Val Tyr Leu Asp Phe Phe Gly
    50                  55                  60

Gly His Gly Ala Leu Val Leu Gly His Gly His Pro Arg Ile Asn Lys
65                  70                  75                  80

Ala Ile Ser Ala Ala Leu Thr His Gly Val Gln Tyr Gly Ala Ser His
                85                  90                  95

Pro Leu Glu Val Arg Trp Ala Glu Arg Leu Val Asn Ala Phe Pro Ser
            100                 105                 110

Met His Lys Val Arg Phe Ala Gly Ser Gly Thr Glu Ala Thr Leu Leu
        115                 120                 125

Ala Leu Arg Met Ala Arg Ala Phe Thr Gly Arg Ser Lys Ile Leu Arg
    130                 135                 140

Ile Ala Thr His Tyr His Gly Trp His Asp Phe Ser Ala Ser Gly Tyr
145                 150                 155                 160

Asn Ser His Phe Asp Gly Gln Pro Ala Pro Gly Val Leu Ala Glu Ile
                165                 170                 175

Ala Gln Ser Thr Leu Leu Val Arg Pro Asp Asp Phe Glu Gly Leu Arg
            180                 185                 190

Ala Leu Phe Ala Gln Tyr Gly Gly Glu Ile Ala Thr Ile Ile Ala Glu
        195                 200                 205

Pro Val Gly Ser His Phe Gly Ile Thr Pro Val Ser Asp Asp Phe Leu
    210                 215                 220

Leu Glu Gly Ala Ala Leu Ala Arg Lys His Gly Ala Ile Phe Ile Leu
225                 230                 235                 240

Asp Glu Val Ile Thr Gly Phe Arg Val Gly Asn His Gly Met Gln Gly
                245                 250                 255

Leu Leu Asp Ile Ala Pro Asp Leu Thr Cys Leu Ala Lys Ala Ser Ala
            260                 265                 270

Gly Gly Leu Pro Gly Gly Val Val Gly Gly Arg Ala Asp Val Met Ala
        275                 280                 285

Val Leu Asp Arg Gly Ser Asp Arg Lys Val Leu His Gln Gly Thr Phe
    290                 295                 300

Thr Gly Asn Pro Ile Thr Ala Ala Ala Ala Ile Ala Ala Ile Asp Thr
305                 310                 315                 320

Ile Ile Glu Asp Asp Val Cys Thr His Ile Asn Ala Leu Gly Gln Tyr
                325                 330                 335

Ala Arg Asp Ser Met Asn Glu Leu Phe Ala Arg Lys His Leu Asp Trp
            340                 345                 350

Leu Ala Tyr Gly Arg Phe Ser Gly Phe His Leu Met Pro Gly Leu Ser
        355                 360                 365

Pro Leu Thr Thr Asp Thr Gly Val Ile Thr Arg Gly Glu Val Ala Arg
    370                 375                 380

Pro Ala Val Lys Met Ile Ala Ala Met Arg Met Ala Leu Ile Leu Glu
385                 390                 395                 400

Gly Ile Asp Ile Gly Gly Arg Gly Ser Val Phe Leu Ser Ala Gln His
                405                 410                 415

Asp Arg Gly His Val Asp Gln Leu Val Ser Thr Phe Asp Ser Ile Leu
            420                 425                 430

Gly Arg Leu Ala Glu Glu Asn Leu Leu Gly
        435                 440

### Annexe 4

### Sequence listings of oligonucleotides

[SEQ ID: No.1]
   gtccacggtc tcccatgcag aatttcgaga t 31
[SEQ ID: No.2]
   acatcccaag cttcacgttc gaggtc 26
[SEQ ID: No.3]
   cgctcggtca tgacgtacgt ttccctg 27
[SEQ ID: No.4]
   acgaagaagc ttatcaaaca acccccag 28
[SEQ ID: No.5]
   atgccgccca gtgacatcgc gtacgc 26
[SEQ ID: No.6]
   ccctcggtac caggtcatcg gccggccact tcc 33
[SEQ ID: No.7]
   atgcgggagc cgctcacgct cgac 24
[SEQ ID: No.8]
   ccaactggta cctggtcatc cgtggctcct gtctcg 36
[SEQ ID: No.11]
   agaattcgcg gcacaggcag gcagcg 26
[SEQ ID: No.12]
   ttataagctt tcagcgctcg gtccggtggc 30
[SEQ ID: No.13]
   tataccatgg cggcacaggc aggc 24
[SEQ ID: No.14]
   ttataagctt gcgctcggtc cggtggc 27
[SEQ ID: No.17]
   agaattcggc gtccgcaact ccgcag 26
[SEQ ID: No.18]
   aataagcttt cagggcgcac ctcgcc 26
[SEQ ID: No.19]
   actcgccaag ggctatggtg tcc 23
[SEQ ID: No.20]
   gccaacagtt ccaacagcgg tgtg 24
[SEQ ID: No.23]
   gtgcacggtc tcgcatgtct atttatagcg attatgaacg taaaac 46
[SEQ ID: No.24]
   gtgcacggtc tcctcgagtt agcccaggag gttttcttca gc 42
[SEQ ID: No.25]
   gggaattcag gaggaattaa ccatgccgcc gagcgac 37
[SEQ ID: No.26]
   gaattcccat attctagaag gtcatcggcc ggccact 37
[SEQ ID: No.27]
   tgggaattca ggaggaatta accatgcag 29
[SEQ ID: No.28]
   cggccaggtc tagatacgtc atcgccg 27
[SEQ ID: No.29]
   tgggtctaga ggaggaatta accatgcgcg agccg 35
[SEQ ID: No.30]
   gaattcccat agcatgcctg gtcatccgtg gctcc 35
[SEQ ID: No.31]
   tttcccaagc ttacaggagg aattaaccat g 31
[SEQ ID: No.32]
   gtaccagctg caaagcttga gttagcccag 30
[SEQ ID: No.33]
   gggtctagag gaggaattaa ccatgagcca gaatctcttt 40
[SEQ ID: No.34]
   ctgcagaacc agcatggtgg tcagtacttc actcatgcg 39
[SEQ ID: No.35]
   gcgtggaagc ttaagaggtt tattatggtt gctgaa 36
[SEQ ID: No.36]
   gtgcacggtc tcgagctgaa ttcttactgg cgattgtcat 40

## Claims

1. A fermentative process in which a recombinant microorganism produces a D-phenylglycine product, in particular D-phenylglycine (D-PG) or D-*p*-hydroxyphenylglycine (D-HPG), wherein
a) for the production of D-PG, respectively for the production of D-HPG, phenylpyruvate (PP), respectively *p*-hydroxyphenylpyruvate (HPP) is withdrawn from the aromatic amino acid pathway
b) and is converted to mandelic acid (MA) or *p*-hydroxymandelic acid (HMA), respectively,
c) thereafter being converted into phenylglyoxylate or *p*-hydroxyphenylglyoxylate, respectively,
d) and the phenylglyoxylate or *p-*hydroxyphenylglyoxylate thereafter being converted into D-phenylglycine (D-PG) or D-*p*-hydroxyphenylglycine (D-HPG) by the action of a stereo-inverting D-aminotransferase, respectively.

2. A process according to claim 1, wherein the microorganism applied provides phenylpyruvate (PP) or *p*-hydroxyphenylpyruvate (HPP) at increased availability.

3. A process according to claim 2, wherein phenylpyruvate (PP) or *p*-hydroxyphenylpyruvate (HPP) is provided at increased availability by feeding phenylalanine (for PP) or tyrosine (for HPP), respectively.

4. A process according to any of claims 1-3, wherein the conversion of phenylpyruvate (PP) to mandelic acid (MA) or the conversion of *p*-hydroxyphenylpyruvate (HPP) to *p*-hydroxymandelic acid (HMA), occurs via a one-step enzymatic reaction catalyzed by a *p*-hydroxymandelate synthase.

5. A process according to any of claims 1-3, wherein the conversion of phenylpyruvate (PP) to mandelic acid (MA) or the conversion of *p*-hydroxyphenylpyruvate (HPP) to *p*-hydroxymandelic acid (HMA) occurs via a multi-step reaction, comprising the steps of converting PP or HPP to phenylacetaldehyde or *p*-hydroxyphenylacetaldehyde, then to phenylacetate or *p*-hydroxyphenylacetate, and finally to MA or HMA, respectively.

6. A process according to any of claims 1-5, wherein the conversion of hydroxymandelic acid (HMA), respectively of mandelic acid (MA), to *p*-hydroxyphenylglyoxylate, respectively to phenylglyoxylate is catalyzed in the presence of an enzyme, selected from a group of enzymes, consisting of mandelate dehydrogenase, *p*-hydroxymandelate dehydrogenase, oxygen dependent mandelate oxidases, and oxygen dependent *p*-hydroxymandelate oxidases.

7. A recombinant cell which is capable of secreting detectible amounts of D-HPG or D-PG and which contains
a) genes coding for enzymes which catalyze the conversion of *p*-hydroxyphenylpyruvate (HPP) to *p*-hydroxymandelic acid (HMA) or phenylpyruvate (PP) to mandelic acid (MA)
b) genes coding for enzymes which catalyze the conversion of *p*-hydroxymandelic acid (HMA) to *p*-hydroxyphenylglyoxylate or of mandelic acid (MA) to phenylglyoxylate
c) and genes coding for a stereo-inverting D-aminotransferase which catalyzes the conversion of *p*-hydroxyphenylglyoxylate to D-HPG or phenylglyoxylate to D-PG.

8. A process according to any of claims 1-6, wherein the process is carried out in *Escherichia coli* as a host microorganism.

9. A recombinant cell according to claim 7, wherein the cell is an *Escherichia coli* cell.

## Patentansprüche

1. Fermentationsverfahren, bei dem ein rekombinanter Mikroorganismus ein D-Phenylglycin-Produkt, insbesondere D-Phenylglycin (D-PG) oder D-*p-*Hydroxyphenylglycin (D-HPG), produziert, wobei
a) zur Produktion von D-PG bzw. zur Produktion von D-HPG Phenylpyruvat (PP) bzw. *p*-Hydroxyphenylpyruvat (HPP) dem aromatischen Aminosäureweg entzogen wird
b) und in Mandelsäure (MA) bzw. *p*-Hydroxymandelsäure (HMA) umgewandelt wird,
c) anschließend in Phenylglyoxylat bzw. *p-*Hydroxyphenylglyoxylat umgewandelt wird
d) und das Phenylglyoxylat oder *p-*Hydroxyphenylglyoxylat danach durch die Einwirkung einer Stereoinversions-D-aminotransferase in D-Phenylglycin (D-PG) bzw. D-*p*-Hydroxyphenylglycin (D-HPG) umgewandelt wird.

2. Verfahren nach Anspruch 1, wobei durch den eingesetzten Mikroorganismus Phenylpyruvat (PP) oder *p*-Hydroxyphenylpyruvat (HPP) mit erhöhter Verfügbarkeit bereitgestellt wird.

3. Verfahren nach Anspruch 2, wobei Phenylpyruvat (PP) oder *p*-Hydroxyphenylpyruvat (HPP) durch Zuführung von Phenylalanin (für PP) bzw. Tyrosin (für HPP) mit erhöhter Verfügbarkeit bereitgestellt wird.

4. Verfahren nach einem der Ansprüche 1-3, wobei die Umwandlung von Phenylpyruvat (PP) in Mandelsäure (MA) oder die Umwandlung von *p*-Hydroxyphenylpyruvat (HPP) in *p*-Hydroxymandelsäure (HMA) über eine von einer *p*-Hydroxymandelat-Synthase katalysierte, aus einem Schritt bestehende enzymatische Reaktion stattfindet.

5. Verfahren nach einem der Ansprüche 1-3, wobei die Umwandlung von Phenylpyruvat (PP) in Mandelsäure (MA) oder die Umwandlung von *p*-Hydroxyphenylpyruvat (HPP) in *p*-Hydroxymandelsäure (HMA) über eine aus mehreren Schritten bestehende Reaktion, bei denen PP bzw. HPP in Phenylacetaldehyd bzw. *p*-Hydroxyphenylacetaldehyd, anschließend in Phenylacetat bzw. *p*-Hydroxyphenylacetat und schließlich in MA bzw. HMA umgewandelt wird, stattfindet.

6. Verfahren nach einem der Ansprüche 1-5, wobei die Umwandlung von Hydroxymandelsäure (HMA) bzw. von Mandelsäure (MA) in *p*-Hydroxyphenylglyoxylat bzw. Phenylglyoxylat in Gegenwart eines Enzyms, ausgewählt aus einer Gruppe von Enzymen, bestehend aus Mandelat-Dehydrogenase, *p*-Hydroxymandelat-Dehydrogenase, sauerstoffabhängige Mandelat-Oxidasen und sauerstoffabhängige *p*-Hydroxymandelat-Oxidasen, katalysiert wird.

7. Rekombinante Zelle, die zur Sekretion nachweisbarer Mengen an D-HPG oder D-PG fähig ist und
a) für Enzyme, die die Umwandlung von *p*-Hydroxyphenylpyruvat (HPP) in *p*-Hydroxymandelsäure (HMA) oder von Phenylpyruvat (PP) in Mandelsäure (MA) katalysieren, codierende Gene,
b) für Enzyme, die die Umwandlung von *p*-Hydroxymandelsäure (HMA) in *p*-Hydroxyphenylglyoxylat oder von Mandelsäure (MA) in Phenylglyoxylat katalysieren, codierende Gene
c) sowie für eine Stereoinversions-D-aminotransferase, die die Umwandlung von *p-*Hydroxyphenylglyoxylat in D-HPG oder von Phenylglyoxylat in D-PG katalysiert, codierende Gene enthält.

8. Verfahren nach einem der Ansprüche 1-6, wobei das Verfahren in *Escherichia coli* als Wirtsmikroorganismus durchgeführt wird.

9. Rekombinante Zelle nach Anspruch 7, bei der es sich um eine *Escherichia-coli*-Zelle handelt.

## Revendications

1. Procédé de fermentation dans lequel un micro-organisme recombinant produit un produit de D-phénylglycine, en particulier la D-phénylglycine (D-PG) ou la D-*p*-hydroxyphénylglycine (D-HPG), dans lequel
a) pour la production de D-PG, respectivement pour la production de D-HPG, du phénylpyruvate (PP), respectivement du *p*-hydroxyphénylpyruvate (HPP) est prélevé de la voie des acides aminés aromatiques
b) et est converti en acide mandélique (MA) ou en acide *p*-hydroxymandélique (HMA), respectivement,
c) étant par la suite converti en phénylglyoxylate ou en *p*-hydroxyphénylglyoxylate, respectivement,
d) et le phénylglyoxylate ou le *p-*hydroxyphénylglyoxylate étant par la suite converti en D-phénylglycine (D-PG) ou en D-*p*-hydroxyphénylglycine (D-HPG) par l'action d'une D-aminotransférase de stéréo-inversion, respectivement.

2. Procédé selon la revendication 1, dans lequel le micro-organisme appliqué fournit du phénylpyruvate (PP) ou du *p*-hydroxyphénylpyruvate (HPP) avec une disponibilité accrue.

3. Procédé selon la revendication 2, dans lequel le phénylpyruvate (PP) ou le *p*-hydroxyphénylpyruvate (HPP) est fourni avec une disponibilité accrue en alimentant en phénylalanine (pour le PP) ou en tyrosine (pour le HPP), respectivement.

4. Procédé selon l'une quelconque des revendications 1 à 3, dans lequel la conversion du phénylpyruvate (PP) en acide mandélique (MA), ou la conversion du *p-*hydroxyphénylpyruvate (HPP) en acide *p-*hydroxymandélique (HMA), se produit par l'intermédiaire d'une réaction enzymatique à une étape catalysée par une *p*-hydroxymandélate synthétase.

5. Procédé selon l'une quelconque des revendications 1 à 3, dans lequel la conversion du phénylpyruvate (PP) en acide mandélique (MA), ou la conversion du *p-*hydroxyphénylpyruvate (HPP) en acide *p-*hydroxymandélique (HMA), se produit par l'intermédiaire d'une réaction à plusieurs étapes, comprenant les étapes de conversion du PP ou du HPP en phénylacétaldéhyde ou en *p*-hydroxyphénylacétaldéhyde, puis en phénylacétate ou en *p*-hydroxyphénylacétate, et finalement en MA ou HMA, respectivement.

6. Procédé selon l'une quelconque des revendications 1 à 5, dans lequel la conversion de l'acide *p-*hydroxymandélique (HMA), respectivement de l'acide mandélique (MA), en *p*-hydroxyphénylglyoxylate, respectivement en phénylglyoxylate, est catalysée en présence d'une enzyme, choisie parmi le groupe d'enzymes constitué par la mandélate déshydrogénase, la *p*-hydroxymandélate déshydrogénase, les mandélate oxydases oxygène-dépendantes, et les *p*-hydroxymandélate oxydases oxygène-dépendantes.

7. Cellule recombinante capable de sécréter des quantités détectables de D-HPG ou de D-PG et qui contient
a) des gènes codant pour des enzymes catalysant la conversion du *p*-hydroxyphénylpyruvate (HPP) en acide *p-*hydroxymandélique (HMA), ou du phénylpyruvate (PP) en acide mandélique (MA),
b) des gènes codant pour des enzymes catalysant la conversion de l'acide *p*-hydroxymandélique (HMA) en *p-*hydroxyphénylglyoxylate ou de l'acide mandélique (MA) en phénylglyoxylate
c) et des gènes codant pour une D-aminotransférase de stéréo-inversion catalysant la conversion du *p-*hydroxyphénylglyoxylate en D-HPG ou du phénylglyoxylate en D-PG.

8. Procédé selon l'une quelconque des revendications 1 à 6, dans lequel le procédé est réalisé dans *Escherichia coli* comme micro-organisme hôte.

9. Cellule recombinante selon la revendication 7, dans laquelle la cellule est une cellule d'*Escherichia coli*.
